(19) Europäisches Patentamt
European Patent Office
Office européen des brevets

(11) Numéro de publication: 0 196 955
B1

(12) # FASCICULE DE BREVET EUROPEEN

(45) Date de publication du fascicule du brevet: 14.06.89

(51) Int. Cl.⁴: C 07 D 487/04, A 61 K 31/55 // (C07D487/04, 223:00, 209:00)

(21) Numéro de dépôt: 86400548.3

(22) Date de dépôt: 14.03.86

(54) **Nouveaux dérivés de la pyrrolo[1,2-a]azépinone, leur préparation et les compositions pharmaceutiques qui les contiennent.**

**Une requête en rectification de la page 9 a été présentée conformément à la règle 88 CBE. Il est statué sur cette requête au cours de la procédure engagée devant la division d'examen.**

(30) Priorité: 15.03.85 FR 8503840

(43) Date de publication de la demande: 08.10.86 Bulletin 86/41

(45) Mention de la délivrance du brevet: 14.06.89 Bulletin 89/24

(84) Etats contractants désignés: AT BE CH DE FR GB IT LI LU NL SE

(56) Documents cités: Néant

(73) Titulaire: RHONE-POULENC SANTE, 20, avenue Raymond Aron, F-92160 Antony (FR)

(72) Inventeur: Barreau, Michel, 24 bis avenue du Clos de Sénart, F-91230 Montgeron (FR)
Inventeur: Comte, Marie-Thérèse, 8 rue de Reims, F-94230 Cachan (FR)
Inventeur: Farge, Daniel, 30 rue des Pins Sylvestres, F-94320 Thiais (FR)
Inventeur: Malleron, Jean-Luc, 5 allée Véronèse Résidence l'Ermitage, F-91940 Les Ulis (FR)
Inventeur: Ponsinet Gérard, 7 rue de Grand Champ, F-94370 Sucy-en-Brie (FR)
Inventeur: Roussel, Gérard, 20 ter rue des Carrières, F-91450 Soisy-sur-Seine (FR)

(74) Mandataire: Le Goff, Yves et al, RHONE-POULENC RECHERCHES Brevets Pharma 25, Quai Paul Doumer, F-92408 Courbevoie (FR)

EP 0 196 955 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne de nouveaux dérivés de la pyrrolo[1,2-a]-azépinone de formule générale:

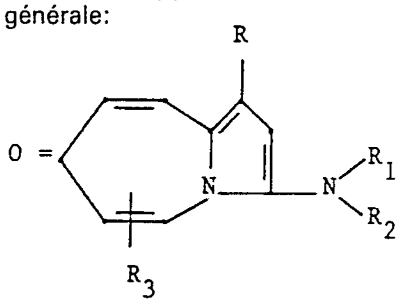

(I)

leurs sels pharmaceutiquement acceptables, leur procédé de préparation ainsi que les compositions pharmaceutiques qui les contiennent.

Dans la formule générale (I), $R_3$ représente un atome d'hydrogène ou d'halogène et

A) soit R représente un radical phénylthio et $R_1$ et $R_2$ forment ensemble avec l'atome d'azote auquel ils sont liés un cycle pipérazine substitué sur le second atome d'azote par un radical alcoyle, alcényle contenant 2 à 4 atomes de carbone,

B) soit R représente un radical phényle éventuellement substitué par un ou plusieurs atomes d'halogène ou radicaux hydroxy, alcoyle, alcoyloxy ou alcoylthio et $R_1$ et $R_2$ forment ensemble avec l'atome d'azote auquel ils sont liés un cycle pipérazine ou homopipérazine substitués sur le second atome d'azote par un radical hydroxyalcoyle dont la partie alcoyle contient 2 à 4 atomes de carbone, alcényle contenant 2 à 4 atomes de carbone, alcynyle contenant 2 à 4 atomes de carbone, benzyle ou phénéthyle dont les portions phényle peuvent être éventuellement substitués par un atome d'halogène ou un radical alcoyle, alcoyloxy, alcoylthio, cyano, trifluorométhyle, carboxy, carboxyalcoyle, alcoyloxycarbonyle, alcoyloxycarbonylalcoyle ou hydroxyalcoyle étant entendu que les radicaux alcoyle et portions alcoyle cités ci-dessus ou qui seront cités par la suite contiennent, sauf mention spéciale, 1 à 4 atomes de carbone en chaîne droite ou ramifiée.

Selon l'invention, les produits de formule générale (I) définis comme précédemment à l'exception de ceux dans la formule desquels $R_1$ et $R_2$ forment ensemble avec l'atome d'azote auquel ils sont liés un cycle pipérazine ou homopipérazine substitués sur le second atome d'azote par un radical hydroxyalcoyle dont la partie alcoyle contient 2 à 4 atomes de carbone, alcynyle contenant 2 à 4 atomes de carbone, benzyle ou phénéthyle substitués par un radical cyano, peuvent être préparés par transposition d'un produit de formule générale:

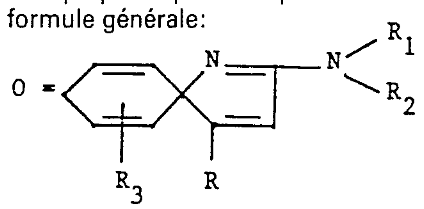

(II)

dans laquelle les symboles R et $R_3$ sont définis comme précédemment et $R_1$ et $R_2$ sont définis comme précédemment à l'exception de former avec l'atome d'azote auquel ils sont liés un cycle pipérazine ou homopipérazine substitués sur le second atome d'azote par un radical hydroxyalcoyle dont la partie alcoyle contient 2 à 4 atomes de carbone, alcynyle contenant 2 à 4 atomes de carbone, benzyle ou phénéthyle substitués par un radical cyano, carboxy, carboxyalcoyle ou hydroxyalcoyle pour obtenir un produit de formule générale (I) dont les symboles sont définis comme précédemment à l'exception pour $R_1$ et $R_2$ de former ensemble avec l'atome d'azote auquel ils sont liés un cycle pipérazine ou homopipérazine substitués sur le second atome d'azote par un radical hydroxyalcoyle dont la partie alcoyle contient 2 à 4 atomes de carbone, alcynyle contenant 2 à 4 atomes de carbone, benzyle ou phénéthyle substitués par un radical cyano, carboxy, carboxyalcoyle ou hydroxyalcoyle, suivie, si on désire obtenir un produit de formule générale (I) dans laquelle R et $R_3$ sont définis comme précédemment et $R_1$ et $R_2$ forment avec l'atome d'azote auquel ils sont liés un cycle pipérazine ou homopipérazine substitués sur le second atome d'azote par un radical benzyle ou phénéthyle dont les parties phényle sont substituées par un radical carboxy, carboxyalcoyle ou hydroxyalcoyle, de la transformation du produit correspondant de formule générale (I) dans laquelle R et $R_3$ sont définis comme précédemment et $R_1$ et $R_2$ forment avec l'atome d'azote auquel ils sont liés un cycle pipérazine ou homopipérazine substitués sur le second atome d'azote par un radical benzyle ou phénéthyle dont les portions phényle sont substituées par un radical alcoyloxycarbonyle ou alcoyloxycarbonylalcoyle, par toute méthode connue pour transformer un ester en acide ou en alcool sans toucher au reste de la molécule.

La transposition du produit de formule générale (II) s'effectue généralement par chauffage à une température comprise entre 190 et 250 °C sans solvant ou dans un solvant organique à haut point d'ébullition tel que le trichloro-1,2,4-benzène.

Les produits de formule générale (II) dans laquelle R et $R_3$ sont définis comme précédemment à l'exception de former avec l'atome d'azote auquel ils sont liés un cycle pipérazine ou homopipérazine substitués sur le second atome d'azote par un radical hydroxyalcoyle dont la partie alcoyle contient 2 à 4 atomes de carbone, alcynyle contenant 2 à 4 atomes de carbone, benzyle ou phénéthyle substitués par un radical cyano, carboxy, carboxyalcoyle ou hydroxyalcoyle peuvent être obtenus par cyclisation d'un produit de formule générale:

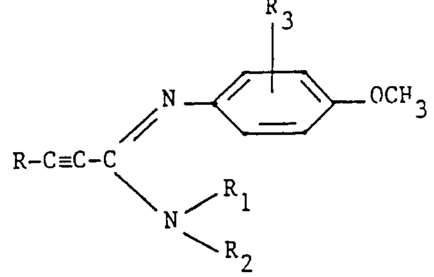

(III)

dans laquelle les symboles R et $R_3$ sont définis comme précédemment et $R_1$ et $R_2$ sont définis comme précédemment à l'exception de former avec l'atome d'azote auquel ils sont liés un cycle pipérazine ou homopipérazine substitués sur le second atome d'azote par un radical hydroxyalcoyle dont la partie alcoyle contient 2 à 4 atomes de carbone, alcynyle contenant 2 à 4 atomes de carbone, benzyle ou phénéthyle substitués par un radical cyano, carboxy, carboxyalcoyle ou hydroxyalcoyle.

La cyclisation s'effectue généralement au moyen d'un acide minéral fort tel que l'acide sulfurique concentré, éventuellement dans un solvant tel que l'acide acétique ou le chloroforme, ou au moyen d'un mélange d'anhydride phosphorique et d'acide méthanesulfonique (1–9 en volumes) à une température comprise entre 0 et 20 °C, ou au moyen d'acide polyphosphorique à une température comprise entre 100 et 120 °C.

Les produits de formule générale (III) peuvent être préparés par action d'un dérivé organométallique de formule générale:

$$R{-}C{\equiv}C{-}Li \qquad (IV)$$

dans laquelle R est défini comme précédemment sur un produit de formule générale:

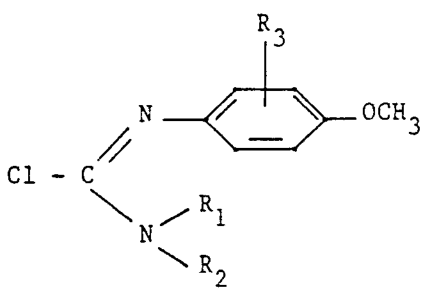
(V)

dans laquelle $R_3$ est défini comme précédemment et $R_1$ et $R_2$ sont définis comme précédemment à l'exception de former avec l'atome d'azote auquel ils sont liés un cycle pipérazine ou homopipérazine substitués sur le second atome d'azote par un radical hydroxyalcoyle dont la partie alcoyle contient 2 à 4 atomes de carbone, alcynyle contenant 2 à 4 atomes de carbone, benzyle ou phénéthyle substitués par un radical cyano, carboxy, carboxyalcoyle ou hydroxyalcoyle.

La réaction s'effectue généralement dans un solvant organique tel qu'un éther comme le tétrahydrofuranne ou un carbure comme l'hexane ou un mélange de ces solvants, à une température comprise entre −70 et +20 °C.

Les produits de formule générale (V) peuvent être préparés par action de la N-(dichlorométhylène)-p-anisidine de formule générale:

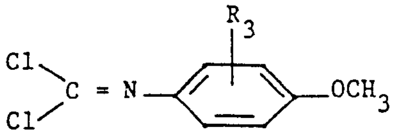
(VI)

dans laquelle $R_3$ est défini comme précédemment sur une amine de formule générale:

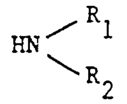
(VII)

dans laquelle $R_1$ et $R_2$ sont définis comme précédemment à l'exception de former avec l'atome d'azote auquel ils sont liés un cycle pipérazine ou homopipérazine substitués sur le second atome d'azote par un radical hydroxyalcoyle dont la partie alcoyle contient 2 à 4 atomes de carbone, alcynyle contenant 2 à 4 atomes de carbone, benzyle ou phénéthyle substitués par un radical cyano, carboxy, carboxyalcoyle ou hydroxyalcoyle.

La réaction s'effectue généralement dans un solvant tel qu'un éther comme le tétrahydrofuranne à une température voisine de 20 °C.

En pratique, il n'est pas nécessaire d'isoler le produit de formule générale (V) ainsi obtenu pour préparer les produits de formule générale (III): Après avoir fair réagir le produit de formule générale (VI) sur le produit de formule générale (VII) comme il vient d'être dit, il suffit d'ajouter le dérivé organo-métallique de formule générale (IV) en quantité suffisante, soit directement dans le mélange réactionnel, soit le cas échéant et si on le désire, après filtration du chlorhydrate d'amine formé.

Les produits de formule générale (VI) peuvent être préparés par application ou adaptation de l'une des méthodes citées par E. Kühle, Ang. Chem. Int. Ed., 1, 647 (1962).

Selon l'invention les produits de formule générale (I) dans laquelle $R_3$ est défini comme précédemment et les autres symboles sont définis comme précédemment en B) peuvent être préparés par action d'un produit de formule générale:

$$R_4X \qquad (VIII)$$

dans laquelle $R_4$ représente un radical hydroxyalcoyle dont la partie alcoyle contient 2 à 4 atomes de cabone, alcényle contenant 2 à 4 atomes de carbone, alcynyle contenant 2 à 4 atomes de carbone, benzyle ou phénéthyle éventuellement substitués par un atome d'halogène ou un radical alcoyle, alcoyloxy, alcoylthio, cyano, trifluorométhyle, carboxy, carboxyalcoyle, alcoyloxycarbonyle, alcoyloxycarbonylalcoyle ou hydroxyalcoyle et X représente un atome d'halogène tel que le chlore, le brome ou l'iode sur un produit de formule générale:

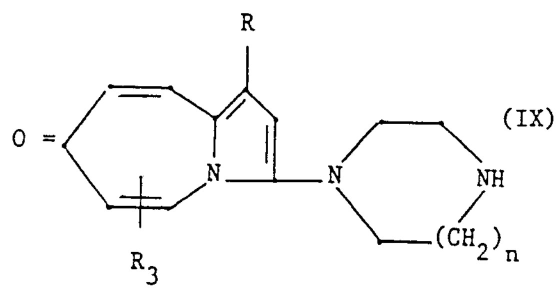
(IX)

dans laquelle n est égal à 1 ou 2 et R et $R_3$ sont définis comme précédemment.

On effectue généralement la réaction dans un solvant organique tel qu'un solvant chloré comme le chloroforme à une température comprise entre 20 °C et la température de reflux du mélange réactionnel, en présence d'un accepteur d'acide tel que la diméthylamino-4-pyridine.

Les produits de formule générale (IX) peuvent être préparés par désallylation d'un produit de formule générale (I) dans laquelle $R_1$ et $R_2$ forment ensemble avec l'atome d'azote auquel ils sont liés un cycle pipérazine ou homopipérazine substitués sur le second atome d'azote par un radical allyle et les autres symboles sont définis comme précédemment, c'est-à-dire un produit de formule générale:

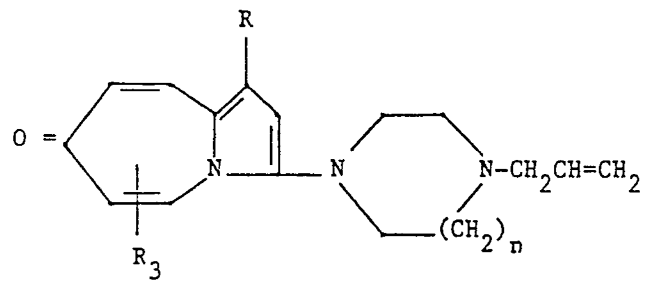

$(I_A)$

La réaction s'effectue généralement dans un solvant hydroorganique tel qu'un mélange de dioxanne et d'eau, à une température comprise entre 50 °C et la température de reflux du mélange réactionnel en présence de trichlorure de rhodium ou d'un catalyseur dérivé de métaux de transition connus pour désallyler les amines comme par exemple ceux qui sont décrits ou cités par D. Pico, M. Cottin, D. Anker et H. Pacheco, Tetr. Letters, 1399 (1983).

Les produits de formule générale ($I_A$) peuvent être préparés par transposition d'un produit de formule générale (II) comme il a été dit précédemment.

Il est évident pour l'homme du métier que, selon la nature des radicaux fixés sur la molécule, il peut être nécessaire de bloquer une fonction particulière avant de mettre en œuvre l'un des procédés selon l'invention. Ainsi lorsque $R_1$ et $R_2$ forment avec l'atome d'azote auquel ils sont liés un cycle pipérazine ou homopipérazine substitués par un radical hydroxyalcoyle, la fonction alcool peut être protégée sous forme de radical tétrahydropyranyloxy que l'on introduit au stade de la synthèse que l'on juge le plus opportun. Le déblocage s'effectue ensuite par tout moyen connu de l'homme du métier, par exemple par action d'un acide minéral aqueux.

Les nouveaux produits de formule générale (I) peuvent être purifiés par les méthodes connues habituelles, par exemple par cristallisation, chromatographie, extractions successives en milieu acide et basique ou formation de sels et recristallisation de ceux-ci.

Les nouveaux produits de formule générale (I) peuvent être transformés en sels d'addition avec les acides par action d'un acide dans un solvant organique tel qu'un alcool une cétone, un éther ou un solvant chloré. Le sel formé précipite, éventuellement après concentration de sa solution; il est séparé par filtration ou décantation.

Lorsque les produits de formule générale (I) possèdent dans leur molécule une fonction acide carboxylique, ils peuvent être transformés en sels métalliques ou en sels d'addition avec les amines en opérant par toute méthode connue.

Les nouveaux produits de formule générale (I) et leurs sels pharmaceutiquement acceptables présentent d'intéressantes propriétés pharmacologiques les rendant utiles comme antipsychotiques. Ils se sont montrés actifs chez le rat à des doses comprises entre 1 et 80 mg/kg par voie orale dans le test d'inhibition des baillements induits par une faible dose d'apomorphine selon I. Dubuc, P. Protais, O. Colboc et J. Costentin, Neuropharmacology, Vol. 21, p. 1203–1206 (1982).

Sont plus particulièrement intéressants, les produits de formule générale (I) dans laquelle $R_3$ représente un atome d'hydrogène ou d'halogène et

A) soit R représente un radical phénylthio et $R_1$ et $R_2$ forment ensemble avec l'atome d'azote auquel ils sont liés un cycle pipérazine substitué par un radical alcoyle, alcényle contenant 2 à 4 atomes de carbone,

B) soit R représente un radical phényle éventuellement substitué par un radical hydroxy, alcoyle ou alcoyloxy et $R_1$ et $R_2$ forment ensemble avec l'atome d'azote auquel ils sont liés un cycle pipérazine ou homopipérazine substitués par un radical hydroxyalcoyle dont la partie alcoyle contient 2 à 4 atomes de carbone, alcényle contenant 2 à 4 atomes de carbone, alcynyle contenant 2 à 4 atomes de carbone, benzyle dont la partie phényle peut être éventuellement substituée par un atome d'halogène ou un radical alcoyle, cyano, trifluorométhyle, carboxy ou alcoyloxycarbonyle.

Sont d'un intérêt tout particulier les produits de formule générale (I) suivants:

– [(méthyl-4 benzyl)-4 pipérazinyl-1]-3 phényl-1 7H-pyrrolo[1,2-a] azépinone-7

– (hydroxy-4 phényl)-1 [(méthyl-4 benzyl)-4 pipérazinyl-1-]-3 7H-pyrrolo[1,2-a] azépinone-7

– [(méthyl-4 benzyl)-4 pipérazinyl-1]-3 (méthoxy-4 phényl)-1 7H-pyrrolo[1,2-a] azépinone-7

– (benzyl-4 pipérazinyl-1)-3 (hydroxy-4 phényl)-1 7H-pyrrolo[1,2-a] azépinone-7

– (benzyl-4 pipérazinyl)-3 (méthoxy-4 phényl)-1

7H-pyrrolo[1,2-a] azépinone-7

– [(fluoro-4 benzyl)-4 pipérazinyl-1]-3 phényl-1 7H-pyrrolo[1,2-a] azépinone-7

– (benzyl-4 pipérazinyl-1 )-3 phényl-1 7H-pyrrolo[1,2-a] azépinone-7

– [(trifluorométhyl-3 benzyl)-4 pipérazinyl-1]-3 phényl-1 7H-pyrrolo[1,2-a] azépinone-7

– [(méthyl-4 benzyl)-4 pipérazinyl-1]-3 (méthoxy-2 phényl)-1 7H-pyrrolo[1,2 a] azépinone-7

– (méthyl-4 pipérazinyl-1)-3 phénylthio-1 7H-pyrrolo[1,2-a] azépinone-7

– (allyl-4 pipérazinyl-1)-3 phénylthio-1 7H-pyrrolo[1,2-a] azépinone-7

– [(carboxy-4 benzyl)-4 pipérazinyl-1]-3 phényl-1 7H-pyrrolo[1,2-a] azépinone-7

Les nouveaux produits de formule générale (I) présentent une toxicité faible. Leur $DL_{50}$ est comprise entre 100 et 900 mg/kg par voie orale chez la souris.

Comme exemples de sels pharmaceutiquement acceptables peuvent être cités les sels d'addition avec les acides minéraux (tels que chlorhydrates, sulfates, nitrates, phosphates) ou organiques (tels que les acétates, propionates, succinates, benzoates, fumarates, maléates, méthanesulfonates, iséthionates, théophyllineacétates, salicylates, phénolphtalinates, méthylène-bis-β-oxynaphtoates) ou des dérivés de substitution de ces composés ou bien les sels avec les métaux alcalins tels que sels de sodium, de potassium ou de lithium ou sels d'addition avec les bases tels que sels d'ammonium, d'éthanolamine ou de lysine.

Les exemples suivants, donnés à titre non limitatif, montrent comment l'invention peut être mise en pratique.

Exemple 1

On chauffe pendant 10 min à une température voisine de 220 °C 40 g d'(allyl-4 pipérazinyl-1)-2 phényl-4 aza-1 spiro[4.5]décatétraène-1,3,6,9 one-8. Après refroidissement à une température voisine de 20 °C, le résidu est dissous dans 500 $cm^3$ de chlorure de méthylène et versé sur 1 kg de silice contenu dans une colonne de 6,5 cm de diamètre. On élue successivement avec 1 l de chlorure de méthylène pur, 1 l d'un mélange de chlorure de méthylène et d'acétate d'éthyle (90–10 en volumes) et 1 l d'un mélange de chlorure de méthylène et d'acétate d'éthyle (80–20 en volumes); les éluats correspondant sont rejetés. On élue ensuite avec 3 l d'un mélange de chlorure de méthylène et d'acétate d'éthyle (70–30 en volumes) et l'éluat correspondant est évaporé à sec sous pression réduite (20 mm de mercure; 2,7 kPa) à 40 °C. On obtient, après recristallisation du résidu dans 200 $cm^3$ d'acétonitrile, 28 g d'(allyl-4 pipérazinyl-1)-3 phényl-1 7H-pyrrolo[1,2-a] azépinone-7 fondant à 125 °C.

L'(allyl-4 pipérazinyl-1)-2 phényl-4 aza-1 spiro[4.5] décatétraène-1,3,6,9 one-8 peut être préparée de la manière suivante: on ajoute en 10 min à une température voisine de 0 °C 200 $cm^3$ d'acide sulfurique concentré sur 49,3 g d'(allyl-4 pipérazinyl-1)-3 méthoxy-4 phénylimino)-3 phényl-1 propyne-1 et agite le mélange réactionnel pendant 12 h à une température voisine de 20 °C. On coule ensuite le mélange réactionnel en 10 min sur 500 g de glace pilée. On ajoute ensuite sous agitation 800 $cm^3$ de soude 10N puis 200 $cm^3$ d'eau distillée. La solution aqueuse est lavée avec 3 fois 500 $cm^3$ de chlorure de méthylène. Les extraits organiques sont réunis, lavés avec 2 fois 200 $cm^3$ d'eau distillée, séchés sur sulfate de magnésium anhydre, filtrés et évaporés à sec sous pression réduite (20 mm de mercure; 2,7 kPa) à 40 °C. Le résidu ainsi obtenu est dissous dans 1 l de chlorure de méthylène et la solution est versée sur 1 kg de silice contenu dans une colonne de 6,2 cm de diamètre. On élue avec 1 l d'un mélange d'acétate d'éthyle et de méthanol (95–5 en volumes) puis 1 l d'un mélange d'acétate d'éthyle et de méthanol (90–10 en volumes); ces éluats sont éliminés. On élue ensuite avec 2 l d'un mélange d'acétate d'éthyle et de méthanol (80–20 en volumes); l'éluat correspondant est évaporé à sec sous pression réduite (20 mm de mercure; 2,7 kPa) à 40 °C. On obtient, après recristallisation du résidu dans 100 $cm^3$ d'acétate d'éthyle, 9,8 g d'(allyl-4 pipérazinyl-1)-2 phényl-4 aza-1 spiro[4.5] décatétraène-1,3,6,9 one-8 fondant à 171 °C.

L'(allyl-4 pipérazinyl-1)-3 (méthoxy-4 phénylamino)-3 phényl-1 propyne-1 peut être préparé de la manière suivante: A une solution de 59 kg de N-(dichlorométhylène) p-anisidine dans 600 $cm^3$ d'éther éthylique, on ajoute à une température voisine de 20 °C et en 20 min une solution de 72 g d'allyl-1 pipérazine dans 450 $cm^3$ de tétrahydrofuranne et poursuit l'agitation pendant encore 30 min à une température voisine de 20 °C. Le précipité qui s'est formé est séparé par filtration. La solution ainsi obtenue est ajoutée en 3 min à une température voisine de –65 °C à une solution de phényléthynyllithium dans 600 $cm^3$ de tétrahydrofuranne obtenue en faisant réagir à une température voisine de 20 °C 30,6 g de phénylacétylène dissous dans 600 $cm^3$ de tétrahydrofuranne avec 187 $cm^3$ d'une solution 1,6M de n-butyllithium dans l'hexane. On laisse réchauffer à une température voisine de 20 °C puis verse le mélange réactionnel sur 500 g de glace pilée. La phase aqueuse est décantée et lavée avec 3 fois 500 $cm^3$ d'éther éthylique. Les fractions éthérées sont réunis, lavées avec 250 $cm^3$ d'eau distillée, séchées sur sulfate de magnésium anhydre, filtrées puis évaporées à sec sous pression réduite (20 mm de mercure; 2,7 kPa) à 40 °C. Le résidu obtenu est dissous dans 200 $cm^3$ de chlorure de méthylène et la solution est versée sur 2 kg de silice contenus dans une colonne de 8 cm de diamètre. On élue avec 4 fois 1 l de mélange de chlorure de méthylène et d'acétate d'éthyle contenant respectivement 10%, 20%, 30% et 40% d'acétate d'éthyle; les éluats correspondants sont éliminés. On élue ensuite avec 3 l d'un mélange de chlorure de méthylène et d'acétate d'éthyle (50–50 en volumes); l'éluat correspondant est évaporé à sec sous pression réduite (20 mm de mercure; 2,7 kPa) à 40 °C. Le résidu obtenu est dissous dans 175 $cm^3$ d'acétonitrile bouillant; on ajoute à la solution 35 g d'acide maléique dissous dans 350 $cm^3$ d'acé-

tonitrile bouillant. Après refroidissement à une température voisine de 20 °C, le précipité formé est séparé par filtration. On obtient ainsi, après recristallisation dans 550 $cm^3$ d'acétonitrile, 58,3 g de dimaléate d'(allyl-4 pipérazinyl-1)-3 (méthoxy-4 phénylimino)-3 phényl-1 propyne-1, fondant à 180 °C.

La N-(dichlorométhylène) p-anisidine peut être préparée selon la méthode décrite par G.M. Dyson et T. Harrington, J. Chem. Soc., 191 (1940).

Exemple 2

Une solution de 3 g de phényl-1 (pipérazinyl-1)-3 7H-pyrrolo[1,2-a] azépinone-7, de 2,5 g d'α-chloro paraxylène et de 1,46 g de diméthylamino-4 pyridine dans 100 $cm^3$ de chloroforme est chauffée pendant 48 h à une température voisine de 60 °C. Après évaporation à sec sous pression réduite (20 mm de mercure; 2,7 kPa) à 40 °C, le résidu obtenu est redissous dans 250 $cm^3$ de chlorure de méthylène et la solution obtenue est versée sur 100 g de silice contenus dans une colonne de 3 cm de diamètre. On élue d'abord avec 1 l de chloroforme pur puis 1 l d'un mélange de chloroforme et de méthanol (95–5 en volumes); ces éluats sont éliminés. On élue ensuite avec 1 l d'un mélange de chloroforme et de méthanol (90–10 en volumes) et l'éluat correspondant est évaporé à sec sous pression réduite (20 mm de mercure; 2,7 kPa) à 40 °C. Le résidu correspondant est dissous dans 10 $cm^3$ d'acétonitrile bouillant. On ajoute goutte à goutte une solution de 0,6 g d'acide oxalique dans 5 $cm^3$ d'éthanol bouillant. Après refroidissement à une température voisine de 20 °C, le précipité formé est séparé par filtration. Après recristallisation du résidu dans une solution de 20 $cm^3$ d'éthanol et 10 $cm^3$ d'eau distillée, on obtient 1,2 g d'oxalate de [(méthyl-4 benzyl)-4 pipérazinyl-1]-3 phényl-1 7H-pyrrolo[1,2-a] azépinone-7, fondant à 150 °C (avec décomposition).

La phényl-1 (pipérazinyl-1)-3 7H-pyrrolo[1,2-a] azépinone-7 peut être préparée de la manière suivante: On chauffe à une température voisine de 88 °C pendant 24 h une solution de 60,8 g d'(allyl-4 pipérazinyl-1)-3 phényl-1 7H-pyrrolo[1,2-a] azépinone-7, de 5,1 g de trichlorure de rhodium et de 9,9 g de diaza-1,4 bicyclo[2.2.2] octane (DABCO) dans 2 l de dioxanne et 200 $cm^3$ d'eau distillée. Après évaporation des solvants sous pression réduite (20 mm de mercure; 2,7 kPa) à 40 °C, le résidu blanc est redissous dans 2 l de chlorure de méthylène et la solution est versée sur 1,5 kg de silice contenus dans une colonne de 7,5 cm de diamètre. On élue d'abord avec 2 l de chloroforme puis 2 l d'un mélange de chloroforme et de méthanol (95–5 en volumes), puis 2 l d'un mélange de chloroforme et de méthanol (90–10 en volumes), puis 2 l d'un mélange de chloroforme et de méthanol (85–15 en volumes). Les éluats correspondants sont éliminés. On élue ensuite avec 4 l d'un mélange de chloroforme et de méthanol (80–20 en volumes) et l'éluat correspondant est évaporé à sec sous pression réduite (20 mm de mercure; 2,7 kPa) à 40 °C. On obtient ainsi 36 g de phényl-1 (pipérazinyl-1)-3 7H-pyrrolo[1,2-a] azépinone-7 sous forme d'une meringue marron utilisée sans autre purification dans les synthèses ultérieures.

L'(allyl-4 pipérazinyl)-3 phényl-1 7H-pyrrolo[1,2-a] azépinone-7 peut être préparée comme décrit à l'exemple 1.

Exemple 3

En opérant comme à l'exemple 2 mais à partir de 3,05 g de phényl-1 (pipérazinyl-1)-3 7H-pyrrolo[1,2-a] azépinone-7, de 2,35 g d'α-bromo p-tolunitrile et de 1,48 g de diméthylamino-4-pyridine en solution dans 80 $cm^3$ de chloroforme, on obtient, après recristallisation dans 25 $cm^3$ d'éthanol, 0,54 g de [(cyano-4 benzyl)-4 pipérazinyl-1]-3 phényl-1 7H-pyrrolo[1,2-a] azépinone-7 fondant à 196 °C.

Exemple 4

On chauffe pendant 10 min à une température voisine de 200 °C, 14,6 g de (benzyl-4-pipérazinyl-1)-2 phényl-4 aza-1-spiro [4.5]-décatétraène-1,3,6,9 one-8. Après refroidissement à une température voisine de 20 °C, le résidu obtenu est dissous dans 500 $cm^3$ de chlorure de méthylène et la solution est versée sur 300 g de silice contenus dans une colonne de 4,2 cm de diamètre. On élue d'abord avec 4 l de chlorure de méthylène pur; l'éluat correspondant est éliminé. On élue ensuite avec 3 l d'un mélange de chlorure de méthylène et de méthanol (90–10 en volumes) et l'éluat est évaporé à sec sous pression réduite (20 mm de mercure; 2,7 kPa) à 40 °C. Après recristallisation du résidu dans 200 $cm^3$ d'acétonitrile, on obtient 10,5 g de (benzyl-4 pipérazinyl-1)-3 phényl-1 7H-pyrrolo[1,2-a] azépinone-7, fondant à 157 °C.

La (benzyl-4 pipérazinyl-1)-2 phényl-4 aza-1 spiro[4.5] décatétraène-1,3,6,9 one-8 peut être préparée de la manière suivante: A 110 $cm^3$ d'acide sulfurique concentré refroidis à une température voisine de 0 °C, on ajoute sous agitation 35,5 g de (benzyl-4 pipérazinyl-1)-3 (méthoxy-4 phénylimino)-3 phényl-1 propyne-1 et agite pendant 12 h en laissant revenir à une température voisine de 20 °C. Le mélange réactionnel est alors coulé dans 500 $cm^3$ d'eau glacée. On ajoute 400 $cm^3$ d'une solution aqueuse de soude 10N et on lave avec 3 fois 1500 $cm^3$ au total de chlorure de méthylène. Les phases organiques sont réunies, séchées sur sulfate de sodium, filtrées et évaporées à sec sous pression réduite (20 mm de mercure; 2,7 kPa) à 40 °C. Le résidu est dissous dans 1000 $cm^3$ de chlorure de méthylène et la solution est versée sur 600 g de silice contenus dans une colonne de 5,4 cm de diamètre. On élue d'abord avec 5 l d'acétate d'éthyle; cet éluat est éliminé. On élue ensuite avec 5 l d'un mélange d'acétate d'éthyle et de méthanol (90–10 en volumes) et l'éluat correspondant est évaporé à sec sous pression réduite (20 mm de mercure; 2,7 kPa) à 40 °C. Après recristallisation dans 250 $cm^3$ d'éthanol, on obtient 20 g de (benzyl-4-pipérazinyl-1)-2 phényl-4 aza-1 spiro-[4.5] décatétraène-1,3,6,9 one-8 fondant à 190 °C.

Le (benzyl-4 pipérazinyl-1)-3 (méthoxy-4 phénylimino)-3 phényl-1 propyne-1 peut être préparé de la manière suivante: A une solution de 51 g de N-(dichlorométhylène) p-anisidine en solution dans 500 $cm^3$ d'éther éthylique, on ajoute à une température voisine de 20 °C et en 20 min une solution de 97 g de benzyl-1 pipérazine dans 300 $cm^3$ de tétrahydrofuranne et poursuit l'agitation pendant encore 30 min à une température voisine de 20 °C. Le précipité qui s'est formé est séparé par filtration. La solution ainsi obtenue est ajoutée en 30 min à une température voisine de −65 °C à une solution de phényléthynyllithium dans 500 $cm^3$ de tétrahydrofuranne obtenue en faisant réagir à une température voisine de −20 °C une solution de 28 g de phénylacétylène dans 500 $cm^3$ de tétrahydrofuranne avec 172 $cm^3$ d'une solution 1,6M de n-butyllithium dans l'hexane. On laisse réchauffer à une température voisine de 20 °C puis verse le mélange réactionnel sur 500 g de glace pilée. La phase aqueuse est décantée et lavée 3 fois avec 1500 $cm^3$ au total d'éther éthylique. Les fractions éthérées sont réunies, lavées avec 250 $cm^3$ d'eau distillée, séchées sur sulfate de magnésium anhydre, filtrées et évaporées à sec sous pression réduite (20 mm de mercure; 2,7 kPa) à 40 °C. Le résidu obtenu est dissous dans 500 $cm^3$ de chlorure de méthylène et la solution est versée sur 2 kg de silice contenus dans une colonne de 8,0 cm de diamètre. On élue d'abord avec 3 l de chlorure de méthylène; l'éluat correspondant est éliminé. On élue ensuite avec 5 l de chlorure de méthylène et l'éluat correspondant est évaporé à sec sous pression réduite (20 mm de mercure; 2,7 kPa) à 40 °C. On obtient ainsi 53 g de (benzyl-4-pipérazinyl-1)-3 (méthoxy-4 phénylimino)-3 phényl-1 propyne-1 fondant à 104 °C.

Exemple 5

On chauffe pendant 4 h à une température voisine de 210 °C une solution de 21,4 g de [(fluoro-4 benzyl)-4 pipérazinyl-1]-2 phényl-4 aza-1 spiro[4.5] décatétraène-1,3,6,9-one-8 dans 500 $cm^3$ de trichloro-1,2,4 benzène. Après évaporation à sec sous pression réduite (1 mm de mercure; 0,13 kPa) à 40 °C, le résidu obtenu est dissous dans 500 $cm^3$ de chloroforme et la solution est versée sur 400 g de silice contenus dans une colonne de 4,7 cm de diamètre. On élue d'abord avec 1 l de chloroforme puis avec 1 l d'un mélange de chloroforme et de méthanol (99–1 en volumes); les éluats correspondants sont éliminés. On élue ensuite avec 2 l d'un mélange de chloroforme et de méthanol (98–2 en volumes) et l'éluat correspondant est évaporé à sec sous pression réduite (20 mm de mercure; 2,7 kPa) à 40 °C. Après recristallisation du résidu dans 180 $cm^3$ d'une solution d'éthanol et de diméthylformamide (85–15 en volumes) on obtient 10,5 g de [(fluoro-4 benzyl-4 pipérazinyl-1]-3 phényl-1 7H-pyrrolo[1,2-a] azépinone-7 fondant à 194 °C.

La [(fluoro-4 benzyl)-4 pipérazinyl-1]-2 phényl-4 aza-1 spiro[4.5] décatétraène-1,3,6,9 one-8 peut être préparée en opérant comme à l'exemple 4 mais à partir de 5,8 g de [(fluoro-4 benzyl)-4 pipérazinyl-1]-3 (méthoxy-4 phénylimino)-3 phényl-1 propyne-1 et de 25 $cm^3$ d'acide sulfurique concentré. Après recristallisation dans 30 $cm^3$ d'acétonitrile, on obtient 2,6 g de [(fluoro-4 benzyl)-4 pipérazinyl-1]-2 phényl-4 aza-1 spiro[4.5] décatétraène-1,3,6,9 one-8 fondant à 188 °C.

Le [(fluoro-4 benzyl)-4 pipérazinyl-1]-3 (méthoxy-4 phénylimino)-3 phényl-1 propyne-1 peut être préparé en opérant comme à l'exemple 4 mais à partir de 20,4 g de N-(dichlorométhylène) p-anisidine en solution dans 150 $cm^3$ d'éther éthylique, de 38,8 g de (fluoro-4-benzyl)-4 pipérazine en solution dans 150 $cm^3$ de tétrahydrofuranne et d'une solution de phényléthynyllithium obtenue en faisant réagir 11,2 g de phénylacétylène en solution dans 200 $cm^3$ de tétrahydrofuranne avec 68 $cm^3$ d'une solution 1,6M de n-butyllithium dans l'hexane. Après recristallisation dans 150 $cm^3$ d'éther isopropylique, on obtient 23 g de [(fluoro-4 benzyl)-4 pipérazinyl-1]-3 (méthoxy-4 phénylimino)-3 phényl-1 propyne-1 fondantà 98 °C.

Exemple 6

En opérant comme à l'exemple 5 mais à partir de 8 g de phényl-4 [(trifluorométhyl-3 benzyl)-4 pipérazinyl-1]-2 aza-1 spiro[4.5] décatétraène-1,3,6,9 one-8 et de 150 $cm^3$ de trichloro-1,2,4 benzène, on obtient, après recristallisation dans 75 $cm^3$ d'éthanol, 2,5 g de phényl-1 [(trifluorométhyl-3 benzyl)-4 pipérazinyl-1]-3 7H-pyrrolo[1,2 a] azépinone-7 fondant à 154 °C.

La phényl-4 [(trifluorométhyl-3 benzyl)-4 pipérazinyl-1]-2 aza-1 spiro[4.5] décatétraène-1,3,6,9 one-8 peut être préparée en opérant comme à l'exemple 4 mais à partir de 6,8 g de (méthoxy-4 phénylimino)-3 phényl-1 [(trifluorométhyl-3 benzyl)-4 pipérazinyl-1]-3 propyne-1 et de 35 $cm^3$ d'acide sulfurique concentré. Après recristallisation dans 120 $cm^3$ d'éther isopropylique, on obtient 2,3 g de phényl-4 [(trifluorométhyl-3 benzyl)-4 pipérazinyl-1]-2 aza-1 spiro[4.5] décatétraène-1,3,6,9 one-8 fondant à 135 °C.

La (méthoxy-4 phénylimino)-3 phényl-1 [(trifluorométhyl-3 benzyl)-4 pipérazinyl-1]-3 propyne-1 peut être préparé en opérant comme à l'exemple 4 mais à partir de 9,4 g de N-(dichlorométhylène)-p-anisidine en solution dans 100 $cm^3$ d'éther éthylique, de 22,5 g de (trifluorométhyl-3 benzyl)-1 pipérazine en solution dans 50 $cm^3$ de tétrahydrofuranne et d'une solution de phényléthynyllithium obtenue en faisant réagir 5,1 g de phénylacéthylène en solution dans 100 $cm^3$ de tétrahydrofuranne avec 31 $cm^3$ d'une solution 1,6M de n-butyllithium dans l'hexane. Le résidu ainsi obtenu est dissous dans 250 $cm^3$ de chloroforme et la solution est versée sur 400 g de silice contenus dans une colonne de 4,7 cm de diamètre. On élue d'abord avec 1 l de chloroforme pur puis 1 l d'un mélange de chloroforme et de méthanol (99–1 en volumes), puis 1 l d'un mélange de chloroforme et de méthanol (98–2 en volumes); les éluats correspondants sont éliminés. On élue ensuite avec 2 l d'un mélange de chloroforme et de méthanol (97,5–2,5 en volumes) et l'éluat correspondant est évaporé à sec sous pression réduite (20 mm de mercure; 2,7 kPa) à 40 °C. Le résidu obtenu est dissous dans 200 $cm^3$ d'éthanol bouillant. A cette solution on ajoute 7,2 g d'acide maléique en solu-

tion dans 150 $cm^3$ d'éthanol bouillant. Après refroidissement à une température voisine de 20 °C, le précipité formé est séparé par filtration, lavé avec 40 $cm^3$ d'éthanol puis 50 $cm^3$ d'éther éthylique. On obtient ainsi 13,6 g de dimaléate de (méthoxy-4 phénylimino)-3 phényl-1[(trifluorométhyl-3 benzyl)-4 pipérazinyl-1]-3 propyne-1, fondant à 175 °C.

Exemple 7

En opérant comme à l'exemple 5 mais à partir de 5,8 g de [(méthyl-2 benzyl)-4 pipérazinyl-1]-2 phényl-4 aza-1 spiro[4.5] décatétraène-1,3,6,9 one-8 et de 50 $cm^3$ de trichloro-1,2,4 benzène, on obtient, après recristallisation dans un mélange d'éther isopropylique et d'acétate d'éthyle (50–50 en volumes), 2,3 g de [(méthyl-2 benzyl)-4 pipérazinyl-1]-3 phényl-1 7H-pyrrolo[1,2-a] azépinone-7 fondant à 133 °C.

La [(méthyl-2 benzyl)-4 pipérazinyl-1]-2 phényl-4 aza-1 spiro[4.5] décatétraène-1,3,6,9 one-8 peut être préparée de la manière suivante: On ajoute par petites portions, en 10 min, 14,2 g de maléate de (méthoxy-4 phénylimino)-3 [(méthyl-2 benzyl)-4 pipérazinyl-1]-3 phényl-1 propyne-1, à 60 $cm^3$ d'acide sulfurique concentré à une température voisine de 0 °C. On agite ensuite pendant 24 h à une température voisine de 20 °C. On verse ensuite le mélange réactionnel sur 200 g de glace pilée et ajoute 130 $cm^3$ de solution aqueuse de soude 10N. La solution aqueuse est décantée et lavée avec 3 fois 500 $cm^3$ de chloroforme. Les fractions chloroformiques sont réunies, séchées sur sulfate de sodium anhydre, filtrées et évaporées à sec sous pression réduite (20 mm de mercure; 2,7 kPa) à 40 °C. Le résidu ainsi obtenu est dissous dans 300 $cm^3$ de chloroforme et cette solution est versée sur 300 g de silice contenus dans une colonne de 4,2 cm de diamètre. On élue d'abord avec 1 l de chloroforme puis 1 l d'un mélange de chloroforme et de méthanol (95–5 en volumes). Ces éluats sont éliminés. On élue ensuite avec 2 l d'un mélange de chloroforme et de méthanol (95–5 en volumes); l'éluat correspondant est évaporé à sec sous pression réduite (20 mm de mercure; 2,7 kPa) à 40 °C. On obtient ainsi 5,8 g de [(méthyl-2 benzyl)-4 pipérazinyl-1]-2 phényl-4 aza-1 spiro[4.5] décatétraène-1,3,6,9 one-8 fondant à 193 °C.

Le (méthoxy-4 phénylimino)-3 [(méthyl-2 benzyl)-4 pipérazinyl-1]-3 phényl-1 propyne-1 peut être préparé en opérant comme à l'exemple 4 mais à partir de 20,4 g de N-(dichlorométhylène)-p-anisidine en solution dans 200 $cm^3$ d'éther éthylique, de 38 g de (méthyl-2-benzyl)-1-pipérazine en solution dans 400 $cm^3$ de tétrahydrofuranne et d'une solution de phényléthynyllithium obtenue en faisant réagir 66 $cm^3$ d'une solution 1,6M de n-butyllithium en solution dans l'hexane sur 10,7 g de phénylacétylène en solution dans 200 $cm^3$ de tétrahydrofuranne. Le maléate est préparé à partir de 8,7 g d'acide maléique. Après recristallisation dans 200 $cm^3$ d'éthanol, on obtient le maléate de (méthoxy-4 phénylimino)-3 [(méthyl-2 benzyl)-4 pipérazinyl-1]-3 phényl-1 propyne-1, fondant à 188 °C.

Exemple 8

En opérant comme à l'exemple 5 mais à partir de 34,8 g de [(méthyl-4 benzyl)-4 pipérazinyl-1]-2 phényl-4 aza-1 spiro[4.5] décatétraène-1,3,6,9 one-8 et de 500 $cm^3$ de trichloro-1,2,4 benzène, on obtient, après recristallisation dans 150 $cm^3$ d'acétonitrile, 12,4 g de [(méthyl-4 benzyl)-4 pipérazinyl-1]-3 phényl-1 7H-pyrrolo[1,2-a] azépinone-7 fondant à 143 °C.

La [(méthyl-4 benzyl)-4 pipérazinyl-1)-2 phényl-4 aza-1 spiro[4.5] décatétraène-1,3,6,9 one-8 peut être préparée en opérant comme à l'exemple 4 mais à partir de 5,2 g de (méthoxy-4 phénylimino)-3 [(méthyl-4 benzyl)-4 pipérazinyl-1]-3 phényl-1 propyne-1 et de 25 $cm^3$ d'acide sulfurique concentré. On obtient ainsi, après recristallisation dans 60 $cm^3$ d'un mélange d'acétonitrile et d'oxyde d'isopropyle (50–50 en volumes), 2,1 g de [(méthyl-4 benzyl)-4 pipérazinyl-1]-2 phényl-4 aza-1 spiro[4.5] décatétraène-1,3,6,9 one-8 fondant à 135 °C.

Le (méthoxy-4 phénylimino)-3 [(méthyl-4 benzyl)-4 pipérazinyl-1]-3 phényl-1 propyne-1 peut être préparé en opérant comme à l'exemple 4 mais à partir de 41 g de N-(dichlorométhylène) p-anisidine en solution dans 200 $cm^3$ d'éther éthylique, de 76 g de (méthyl-4 benzyl)-1 pipérazine en solution dans 450 $cm^3$ de tétrahydrofuranne et d'une solution de phényléthynyllithium obtenue en faisant réagir 21,5 g de phénylacétylène en solution dans 400 $cm^3$ de tétrahydrofuranne avec 130 $cm^3$ d'une solution 1,6M de n-butyllithium dans l'hexane. Le résidu ainsi obtenu est dissous dans 400 $cm^3$ d'éthanol bouillant. On ajoute à la solution 37,3 g d'acide maléique en solution dans 400 $cm^3$ d'éthanol bouillant. Après refroidissement à une température voisine de 20 °C, le précipité formé est séparé par filtration, puis recristallisé dans 1600 $cm^3$ d'éthanol. On obtient ainsi 67,7 g de maléate de (méthoxy-4 phénylimino)-3 [(méthyl-4 benzyl)-4 pipérazinyl-1]-3 phényl-1 propyne-1, fondant à 182 °C.

Exemple 9

En opérant comme à l'exemple 5 mais à partir de 3,2 g de (méthyl-4 pipérazinyl-1)-2 phénylthio-4 aza-1 spiro[4.5] décatétraène-1,3,6,9 one-8 et de 100 $cm^3$ de trichloro-1,2,4 benzène, on obtient, après recristallisation dans 20 $cm^3$ d'oxyde d'isopropyle, 0,9 g de (méthyl-4 pipérazinyl-1)-3 phénylthio-1 7H-pyrrolo[1,2-a] azépinone-7 fondant à 120 °C.

La (méthyl-4 pipérazinyl-1)-2 phénylthio-4 aza-1 spiro[4.5] décatétraène-1,3,6,9 one-8 on peut être préparée en opérant comme à l'exemple 4 mais à partir de 7 g de (méthyl-4 pipérazinyl-1)-3 (méthoxy-4 phénylimino)-3 phénylthio-1 propyne-1 et de 50 $cm^3$ d'acide sulfurique concentré. On obtient ainsi, après recristallisation dans 80 $cm^3$ d'un mélange d'acétate d'éthyle et d'oxyde d'isopropyle (50–50 en volumes) 1,1 g de (méthyl-4 pipérazinyl-1)-2 phénylthio-4 aza-1 spiro[4.5] décatétraène-1,3,6,9 one-8 fondant à 142 °C.

Le (méthoxy-4-phénylimino)-3 (méthyl-4 pipérazinyl-1)-3 phénylthio-1 propyne-1 peut être préparé en opérant comme à l'exemple 4 mais à partir de 10,2 g de N-(dichlorométhylène) p-anisidine en solution dans 50 $cm^3$ d'éther éthylique, de 10 g de méthyl-4 pipérazine en solution dans 50 $cm^3$ de tétrahydrofuranne et d'une solution de phénylthioéthynyllithium obtenue en faisant réagir 6,7 g de phénylthioacétylène en solution dans 50 $cm^3$ de tétrahydrofuranne avec 31 $cm^3$ d'une solution 1,6M de n-butyllithium dans l'hexane. Le résidu ainsi obtenu est dissous dans 100 $cm^3$ d'éthanol bouillant. On ajoute à cette solution 8,3 g d'acide maléique en solution dans 150 $cm^3$ d'éthanol bouillant. Après refroidissement à une température voisine de 20 °C, le précipité formé est séparé par filtration et recristallisé dans 350 $cm^3$ d'éthanol. On obtient ainsi 10,3 g de (méthoxy-4 phénylimino)-3 (méthyl-4 pipérazinyl-1)-3 phénylthio-1 propyne-1 fondant à 160 °C.

Le phénylthioacétylène peut être préparé selon la méthode décrite par R.C. Cookson et R. Gopalan, J. Chem. Soc. Chem. Comm., 924 (1978).

Exemple 10

En opérant comme à l'exemple 5 mais à partir de 10,5 g de chloro-7 [(méthyl-4 benzyl)-4 pipérazinyl-1]-2 phenyl-4 aza-1 spiro[4.5] décatétraène-1,3,6,9 one-8 et de 200 $cm^3$ de trichloro-1,2,4 benzène, on obtient un résidu que l'on redissout dans 60 $cm^3$ d'acétonitrile bouillant. A cette solution, on ajoute 3,3 g d'acide maléique en solution dans 60 $cm^3$ d'acétonitrile bouillant. Après refroidissement à une température voisine de 20 °C, la solution obtenue est évaporée à sec sous pression réduite (20 mm de mercure; 2,7 kPa) à 40 °C. Après recristallisation du résidu dans un mélange de 50 $cm^3$ de méthyléthylcétone et 10 $cm^3$ d'oxyde d'isopropyle, on obtient 2,3 g de chloro-6 [(méthyl-4 benzyl)-4 pipérazinyl-1]-3 phényl-1 7H-pyrrolo[1,2-a] azépinone-7 fondant à 183 °C.

La chloro-7 [(méthyl-4 benzyl)-4 pipérazinyl-1]-2 phényl-4 aza-1 spiro[4.5] décatétraène-1,3,6,9 one-8 peut être préparée en opérant comme à l'exemple 4 mais à partir de 31,6 g de (chloro-3 méthoxy-4 phénylimino)-3 [(méthyl-4 benzyl)-4 pipérazinyl-1]-3 phényl-1 propyne-1 et de 150 $cm^3$ d'acide sulfurique concentré. Après recristallisation du résidu dans 220 $cm^3$ d'éthanol, on obtient 7,3 g de chloro-7 [(méthyl-4 benzyl)-4 pipérazinyl-1]-2 phényl-4 aza-1 spiro[4.5] décatétraène-1,3,6,9 one-8 fondant à 161 °C.

Le (chloro-3 méthoxy-4 phénylimino)-3 [(méthyl-4 benzyl)-4 pipérazinyl-1]-3 phényl-1 propyne-1 peut être préparé en opérant comme à l'exemple 4 mais à partir de 44,8 g de N-(dichlorométhylène) chloro-3 méthoxy-4 aniline en solution dans 375 $cm^3$ d'éther éthylique, de 71,5 g de (méthyl-4 benzyl)-4 pipérazine en solution dans 188 $cm^3$ de tétrahydrofuranne et d'une solution de phényléthynyllithium obtenue en faisant réagir 23,97 g de phénylacétylène en solution dans 470 $cm^3$ de tétrahydrofuranne avec 141 $cm^3$ d'une solution 1,6M de n-butyllithium dans l'hexane. On obtient ainsi, après recristallisation dans 45 $cm^3$ d'acétonitrile, 5 g de (chloro-3 méthoxy-4 phénylimino)-3[(méthyl-4 benzyl)-4 pipérazinyl-1]-3 phényl-1 propyne-1 fondant à 113 °C.

Exemple 11

En opérant comme à l'exemple 5 mais à partir de 13 g de (benzyl-4 pipérazinyl-1)-2 (méthoxy-4 phényl)-4 aza-1 spiro[4.5] décatétraène-1,3,6,9 one-8 et de 250 $cm^3$ de trichloro-1,2,4 benzène, on obtient, après recristallisation dans 150 $cm^3$ d'acétonitrile, 8,7 g de (benzyl-4 pipérazinyl-1)-3 méthoxy-4 phényl)-1 7H-pyrrolo[1,2-a] azépinone-7 fondant à 155 °C.

La (benzyl-4 pipérazinyl-1)-2 (méthoxy-4 phényl)-4 aza-1 spiro[4.5] décatétraène-1,3,6,9 one-8 peut être préparée de la manière suivante: A une solution agitée de 13 g de (benzyl-4 pipérazinyl-1)-3 (méthoxy-4 phényl)-1 (méthoxy-4 phénylimino)-3 propyne-1 dans 60 $cm^3$ d'acide acétique, on ajoute en 5 min à une température voisine de 15 °C 10 $cm^3$ d'acide sulfurique concentré et laisse agiter pendant 48 h à une température voisine de 20 °C. Le mélange réactionnel est ensuite évaporé à sec sous pression réduite (20 mm de mercure; 2,7 kPa) à 40 °C. Le résidu obtenu est repris avec 40 $cm^3$ d'une solution d'ammoniaque 11N et le mélange est extrait avec 3 fois 250 $cm^3$ de chlorure de méthylène. Les fractions chlorométhyléniques sont réunis, lavées avec 2 fois 100 $cm^3$ d'eau distillée, séchées sur sulfate de magnésium anhydre, filtrées et évaporées à sec sous pression réduite (20 mm de mercure; 2,7 kPa) à 40 °C. On obtient ainsi 13 g de (benzyl-4 pipérazinyl-1)-2 (méthoxy-4 phényl)-4 aza-1 spiro[4.5] décatétraène-1,3,6,9 one-8 fondant à 190 °C.

Le (benzyl-4 pipérazinyl-1)-3 (méthoxy-4 phénylimino)-3 (méthoxy-4 phényl)-1 propyne-1 peut être préparé de la manière suivante: A une solution de 41 g de N-(dichlorométhylène) p-anisidine dans 450 $cm^3$ d'éther éthylique, on ajoute à une température voisine de 5 °C et en 20 min une solution de 71 g de benzyl-4 pipérazine dans 500 $cm^3$ de tétrahydrofuranne et agite pendant 2 h à une température voisine de 20 °C. L'insoluble qui s'est formé est séparé par filtration. La solution ainsi obtenue est ajoutée en 30 min à une température voisine de −65 °C à une solution de (méthoxy-4 phényl)-2 éthynyllithium obtenue en ajoutant en 20 min, à une température voisine de −70 °C, 310 $cm^3$ d'une solution, 1,6M de n-butyllithium dans l'hexane à une solution de 73 g de méthoxy-4-β,β-dibromostyrène dans 750 $cm^3$ de tétrahydrofuranne, en laissant revenir ensuite à une température voisine de 20 °C et en agitant encore pendant 1 h à cette température.

On laisse revenir le mélange réactionnel à une température voisine de 20 °C et on agite pendant 12 h à cette température; ensuite le mélange réactionnel est versé sur 1 kg de glace pilée. La solution aqueuse est extraite avec 1 l d'éther éthylique puis 2 fois 500 $cm^3$ de chlorure de méthylène. Les fractions organiques sont réunies, lavées avec 250

$cm^3$ d'eau distillée, séchées sur sulfate de sodium anhydre, filtrées et évaporées à sec sous pression réduite (20 mm de mercure; 2,7 kPa) à 40 °C. Le résidu ainsi obtenu est agité pendant 10 min à une température voisine de 20 °C avec 800 $cm^3$ d'acétate d'éthyle. Le résidu qui ne s'est pas dissous est séparé par filtration. On obtient ainsi, après recristallisation dans 550 $cm^3$ d'acétonitrile, 25 g de (benzyl-4 pipérazinyl-1)-3 (méthoxy-4 phénylimino)-3 (méthoxy-4 phényl)-1 propyne-1 fondant à 161 °C.

Le méthoxy-4 β,β-dibromostyrène peut être préparé par la méthode décrite par H.J. Bestmann et K. Li, Chem. Ber., 115, 828 (1982).

Exemple 12

En opérant comme à l'exemple 5 mais à partir de 3,8 g de (benzyl-4 pipérazinyl-1)-2 (hydroxy-4 phényl)-4 aza-1 spiro[4.5] décatétraène-1,3,6,9 one-8 et de 100 $cm^3$ de trichloro-1,2,4 benzène, on obtient, après recristallisation dans 30 $cm^3$ d'un mélange d'acétonitrile et de diméthylformamide (70–30 en volumes), 1,9 g de (benzyl-4 pipérazinyl-1)-3 (hydroxy-4 phényl)-1 7H-pyrrolo[1,2-a] azépinone-7 fondant à 186 °C.

La (benzyl-4 pipérazinyl-1)-2 (hydroxy-4 phényl)-4 aza-1 spiro[4.5] décatétraène-1,3,6,9 one-8 peut être obtenue de la manière suivante: On chauffe pendant 12 h à une température voisine de 100 °C 6,3 g de (benzyl-4 pipérazinyl-1)-2 (méthoxy-4 phényl)-4 aza-1 spiro[4.5] décatétraène-1,3,6,9 one-8 en solution dans 150 $cm^3$ d'acide acétique et 100 $cm^3$ d'une solution d'acide bromhydrique à 48%. Aprés refroidissement à une température voisine de 20 °C, la solution est évaporée à sec sous pression réduite (20 mm de mercure; 2,7 kPa) à 40 °C. On ajoute au résidue 200 g de glace pilée puis 500 $cm^3$ d'une solution aqueuse d'ammoniaque 11N. La solution aqueuse est lavée avec 2 fois 300 $cm^3$ de chloroforme. Les fractions organiques sont réunies, lavées avec 200 $cm^3$ d'eau distillée, séchées sur sulfate de sodium anhydre, filtrées et évaporées à sec sous pression réduite (20 mm de mercure; 2,7 kPa) à 40 °C. Le résidu obtenue est dissous dans 200 $cm^3$ de chloroforme et la solution est versée sur 120 g de silice contenus dans une colonne de 3,2 cm de diamètre. On élue d'abord avec 1 l de chloroforme et l'éluat correspondant est éliminé. On élue ensuite avec 1 l d'un mélange de chloroforme et de méthanol (95–5 en volumes) et l'éluat correspondant est évaporé à sec sous pression réduite (20 mm de mercure; 2,7 kPa) à 40 °C. On obtient ainsi 3,8 g de (benzyl-4 pipérazinyl-1)-2 (hydroxy-4 phényl)-4 aza-1 spiro[4.5] décatétraène-1,3,6,9 one-8, sous forme d'une meringue marron.

Rf = 0,19 (chromatographie sur couche mince de silice; éluant: chloroforme-méthanol (90–10 en volumes).

Spectre de masse: $m/z = 411$ ($M^{+}$).

La (benzyl-4 pipérazinyl-1)-2 (méthoxy-4 phényl)-4 aza-1 spiro[4.5] décatétraène-1,3,6,9 one-8 peut être préparée comme décrit à l'exemple 11.

Exemple 13

En opérant comme à l'exemple 5 mais à partir de 15,2 g d'(allyl-4 pipérazinyl-1)-2 phénylthio-4 aza-1 spiro[4.5] décatétraène-1,3,6,9 one-8 et de 150 $cm^3$ de trichloro-1,2,4 benzène, on obtient un résidu que l'on redissout dans 300 $cm^3$ chloroforme. La solution obtenue est versée sur 300 g de silice contenus dans une colonne de 4,2 cm de diamètre. On élue d'abord avec 1 l de chloroforme et cet éluat est éliminé. On élue ensuite avec 3 l d'un mélange de chloroforme et de méthanol (90–10 en volumes) et l'éluat correspondant est évaporé à sec sous pression réduite (20 mm de mercure; 2,7 kPa) à 40 °C. Le résidu obtenu est dissous dans 15 $cm^3$ d'acétone. A la solution obtenue, on ajoute 1,9 g d'acide oxalique en solution dans 15 $cm^3$ d'acétone. Le précipité formé est séparé par filtration et lavé avec 10 $cm^3$ d'éther éthylique. On obtient ainsi 2,45 g de dioxalate d'(allyl-4 pipérazinyl-1)-3 phénylthio-1 7H-pyrrolo[1,2-a] azépinone-7, fondant à 225 °C.

L'(allyl-4 pipérazinyl-1)-2 phénylthio-4 aza-1 spiro[4.5] décatétraène-1,3,6,9 one-8 peut être préparée en opérant comme à l'exemple 4 mais à partir de 25 g d'(allyl-4 pipérazinyl-1)-3 (méthoxy-4 phénylimino)-3 phénylthio-1 propyne-1 et de 125 $cm^3$ d'acide sulfurique concentré. On obtient ainsi 15,2 g d'(allyl-4 pipérazinyl-1)-2 phénylthio-4 aza-1 spiro[4.5] décatétraène-1,3,6,9 one-8 fondant à 175 °C.

L'(allyl-4 pipérazinyl-1)-3 (méthoxy-4 phénylimino)-3 phénylthio-1 propyne-1 peut être préparé en opérant comme à l'exemple 4 mais à partir de 54 g de N-(dichlorométhylène) p-anisidine en solution dans 500 $cm^3$ d'éther éthylique, de 67 g d'allyl-4 pipérazine en solution dans 450 $cm^3$ d'éther éthylique et d'une solution de phénylthioéthynyllithium obtenue en faisant réagir 34,4 g de phénylthioacétylène en solution dans 250 $cm^3$ de tétrahydrofuranne avec 154 $cm^3$ d'une solution 1,6M de n-butyllithium dans l'hexane. On obtient ainsi, après recristallisation dans 150 $cm^3$ d'acétonitrile, 32 g d'(allyl-4 pipérazinyl-1)-3 (méthoxy-4 phénylimino)-3 phénylthio-1 propyne-1 fondant à 75 °C.

Exemple 14

A une solution de 3 g de (pipérazinyl-1)-3 phényl-1 7H-pyrrolo[1,2-a] azépinone-7 et de 1,46 g de diméthylamino-4 pyridine dans 100 $cm^3$ de chloroforme, on ajoute 1,45 g de bromure de propargyle en solution dans 5 $cm^3$ de chloroforme. La solution est chauffée sous agitation pendant 12 h à une température voisine de 55°C. Après refroidissement à une température voisine de 20 °C, le mélange réactionnel est évaporé à sec sous pression réduite (20 mm de mercure; 2,7 kPa) à 40 °C et le résidu obtenu est dissous dans 100 $cm^3$ de chlorure de méthylène. Cette solution est versée sur 80 g de silice contenus dans une colonne de 2,7 cm de diamètre. On élue d'abord avec 200 $cm^3$ de chloroforme; l'éluat correspondant est éliminé. On élue ensuite avec 500 $cm^3$ d'un mélange de chloroforme et de méthanol (95–5 en volumes); l'éluat correspondant est évaporé à sec sous pression réduite (20 mm de mercure; 2,7 kPa) à 40 °C. Le résidu obtenu est dissous dans 20 $cm^3$ d'acétonitrile. On ajoute à cette solution 690 mg d'acide fumarique en solution dans 5 $cm^3$ d'éthanol bouillant. Après refroidissement à une température voisine de 0 °C, le précipité qui s'est formé est séparé par filtration. On obtient, après recristallisation dans 12 $cm^3$ d'acétonitrile bouillant, 0,40 g de fumarate de (propargyl-4 pipérazinyl-1)-3 phényl-1 7H-pyrrolo[1,2-a] azépinone-7, fondant à 202 °C.

La (pipérazinyl-1)-3 phényl-1 7H-pyrrolo[1,2-a] azépinone-7 peut être préparé comme décrit à l'exemple 2.

Exemple 15

A une solution de 1,5 g de phényl-1 [(tétrahydropyranyloxy-2 éthyl)-4 pipérazinyl-1]-3 7H-pyrrolo[1,2-a] azépinone-7 dans 15 $cm^3$ de chloroforme et 15 $cm^3$ de méthanol on ajoute 1 $cm^3$ d'une solution aqueuse d'acide chlorhydrique 11,5M et agite à une température voisine de 20 °C pendant 12 h. Les solvants sont évaporés et le résidu ainsi obtenu est dissous dans 50 $cm^3$ de chlorure de méthylène. La solution est lavée avec 25 $cm^3$ d'une solution aqueuse de soude 1N et 2 fois avec 50 $cm^3$ d'eau distillée au total, séchée sur sulfate de magnésium anhydre en présence de noir décolorant, filtrée et évaporée à sec sous pression réduite (20 mm de mercure; 2,7 kPa) à 40 °C. On obtient ainsi 0,28 g d'[(hydroxy-2 éthyl)-4 pipérazinyl-1]-3 phényl-1 7H-pyrrolo[1,2-a] azépinone-7, sous forme d'une huile jaune pâle.

Spectre de RMN (250 MHz, $CDCl_3$, δ en ppm, J en Hz):

2,7: triplet, 2H : > N-$C\underline{H}_2$-
2,8: singulet, 4H
3,10: triplet, 4H

-$CH_2$- de la pipérazine

3,70: multiplet, 3H: -$CH_2OH$
5,95: double doublet: J = 11 et 2
6,15: double doublet: J = 12,5 et 2

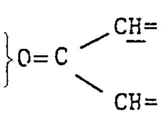

6,25: singulet, 1H: -C$\underline{H}$= du pyrrole
7,30: doublet, 1H, J = 12,5

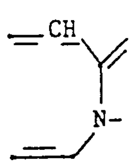

ou

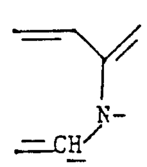

7,45: massif, 5H: protons aromatiques
7,65: doublet, 1H, J = 11

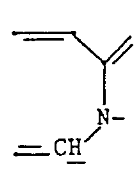

ou

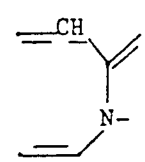

La phényl-1[(tétrahydropyranyloxy-2 éthyl)-4 pipérazinyl-1]-3 7H-pyrrolo[1,2-a] azépinone-7 peut être préparée en opérant comme à l'exemple 2 mais à partir de 6,8 g de phényl-1 (pipérazinyl-1)-3 7H-pyrrolo[1,2-a] azépinone-7, de 5,12 g de bromo-1 tétrahydropyranyloxy-2 éthane et de 3 g de diméthylamino-4 pyridine dans 150 $cm^3$ de chloroforme. Après évaporation à sec sous pression réduite (20 mm de mercure; 2,7 kPa) à 40 °C le résidu obtenu est dissous dans 150 $cm^3$ de chloroforme et la solution est versée sur 500 g de silice contenus dans une colonne de 5 cm de diamètre. On élue d'abord avec 1 l de chloroforme puis avec 1 l d'un mélange de chloroforme et de méthanol (98–2 en volumes); les éluats correspondants sont éliminés. On élue ensuite avec 2 l d'un mélange de chloroforme et de méthanol (96–4 en volumes); l'éluat correspondant est évaporé à sec sous pression réduite (20 mm de mercure; 2,7 kPa) à 40 °C. On obtient ainsi 1,5 g de phényl-1 [(tétrahydropyranyloxy-2 éthyl)-4 pipérazinyl-1]-3 7H-pyrrolo-[1,2-a] azépinone-7 sous forme d'une huile orange employée telle quelle dans la synthèse ultérieure.

Le bromo-1 tétrahydropyranyloxy-2 éthane peut être préparé selon la méthode décrite par W.E. Parham et E.L. Anderson, J. Am. Chem. Soc., 70, 4187 (1948).

La (pipérazinyl-1)-3 phényl-1 7H-pyrrolo[1,2-a] azépinone-7 peut être preparée comme décrit à l'exemple 2.

Exemple 16

En opérant comme à l'exemple 5 mais à partir de 2,6 g de [(méthyl-4 benzyl)-4 homopipérazinyl-1]-2 phényl-4 aza-1 spiro[4.5] décatétraène-1,3,6,9 one-8 et de 55 $cm^3$ de trichloro-1,2,4 benzène, on obtient 2,5 g d'huile que l'on redissout dans 10 $cm^3$ d'acétate d'éthyle. A la solution obtenue, on ajoute 0,70 g d'acide maléique; le produit qui cristallise est séparé par filtration et lavé avec 25 $cm^3$ d'éther éthylique. On obtient ainsi 2 g de maléate de [(méthyl-4 benzyl)-4 homopipérazinyl-1]-3 phényl-1 7H-pyrrolo[1,2-a] azépinone-7, fondant à 165 °C.

La [(méthyl-4 benzyl)-4 homopipérazinyl-1]-2 phényl-4 aza-1 spiro[4.5] décatétraène-1,3,6,9 one-8 peut être préparée en opérant comme à l'exemple 4 mais à partir de 7 g de (méthoxy-4 phénylimino)-3 [(méthyl-4 benzyl)-4 homopipérazinyl-1]-3 phényl-1 propyne-1 et de 35 $cm^3$ d'acide sulfurique concentré. Après recristallisation dans un mélange d'oxyde d'isopropyle et d'acétonitrile (50–50 en volumes) on obtient 1,8 g de [(méthyl-4 benzyl)-4 homopipérazinyl-1]-2 phényl-4 aza-1 spiro[4.5] décatétraène-1,3,6,9 one-8 fondant à 122 °C.

Le (méthoxy-4 phénylimino)-3 [(méthyl-4 benzyl)-4 homopipérazinyl1]-3 phényl-1 propyne-1 peut être préparé en opérant comme à l'exemple 4 mais à partir de 12 g de N-(dichlorométhylène) p-anisidine en solution dans 120 $cm^3$ d'éther éthylique, de 23,4 g de (méthyl-4 benzyl)-1 homopipérazine en solution dans 25 $cm^3$ de tétrahydrofuranne et d'une solution de phényléthynyllithium obtenue en faisant réagir 6,2 g de phénylacétylène en solution dans 100 $cm^3$ de tétrahydrofuranne avec 38 $cm^3$ d'une solution 1,6M de n-butyllithium dans

l'hexane. Après recristallisation dans 50 $cm^3$ d'éther de pétrole, on obtient 4,8 g de (méthoxy-4 phénylimino)-3 [(méthyl-4 benzyl)-4 homopipérazinyl-1]-3 phényl-1 propyne-1 fondant à 66 °C.

Exemple 17

En opérant comme à l'exemple 5 mais à partir de 3,9 g de (méthoxy-4 phényl)-4 [(méthyl-4 benzyl)-4 pipérazinyl-1]-2 aza-1 spiro[4.5] décatétraène-1,3,6,9 one-8 et de 100 $cm^3$ de trichloro-1,2,4 benzène, on obtient, après recristallisation dans 30 $cm^3$ de butanone-2, 2,3 g de (méthoxy-4 phényl)-1 [(méthyl-4 benzyl)-4 pipérazinyl-1]-3 7H-pyrrolo[1,2-a] azépinone-7 fondant à 153 °C.

La (méthoxy-4 phényl-4 [(méthoxy-4 benzyl)-4 pipérazinyl-1]-2 aza-1 spiro[4.5] décatétraène-1,3,6,9 one-8 peut être préparé comme à l'exemple 11 mais à partir de 5,1 g de (méthoxy-4 phényl)-1 (méthoxy-4 phénylimino)-3 [(méthyl-4 benzyl)-4 pipérazinyl-1]-3 propyne-1, 50 $cm^3$ d'acide acétique et 2 $cm^3$ d'acide sulfurique concentré. On obtient ainsi, après recristallisation dans 30 $cm^3$ d'éther isopropylique, 3,9 g de (méthoxy-4 phényl)-4 [(méthoxy-4 benzyl)-4 pipérazinyl-1]-2 aza-1 spiro[4.5] décatétraène-1,3,6,9 one-8, fondant à 142 °C.

Le (méthoxy-4 phényl)-1 (méthoxy-4 phénylimino)-3 [(méthyl-4 benzyl)-4 pipérazinyl-1]-3 propyne-1 peut être préparé comme à l'exemple 11 mais à partir de 4 g de N-(dichlorométhylène) p-anisidine, 7,6 g de (méthyl-4 benzyl)-1 pipérazine, 16 $cm^3$ de sulution, 1,6M de n-butyllithium dans l'hexane et 5,8 g de méthoxy-4 β,β-dibromostyrène. On obtient, après recristallisation dans 50 $cm^3$ d'acétonitrile, 5,1 g de (méthoxy-4 phényl)-1 (méthoxy-4 phénylimino)-3 [(méthyl-4 benzyl)-4 pipérazinyl-1]-3 propyne-1 fondant à 107 °C.

Exemple 18

En opérant comme à l'exemple 5 mais à partir de 2,8 g d'(hydroxy-4 phényl)-4 [(méthyl-4 benzyl)-4 pipérazinyl-1]-2 aza-1 spiro[4.5] décatétraène-1,3,6,9 one-8 et de 80 $cm^3$ de trichloro-1,2,4 benzène, on obtient, après recristallisation dans 40 $cm^3$ de butanone-2, 1,3 g d'(hydroxy-4 phényl)-1 [(méthyl-4 benzyl)-4 pipérazinyl-1]-3 7H-pyrrolo[1,2 a] azépinone-7 fondant à 206 °C.

L'(hydroxy-4 phényl)-4 [(méthyl-4 benzyl)-4 pipérazinyl-1]-2 aza-1 spiro[4.5] décatétraène-1,3,6,9 one-8 peut être obtenue de la manière suivante: on chauffe pendant 8 h à une température voisine de 100 °C 8,1 g de (méthoxy-4 phényl)-1 [(méthyl-4 benzyl)-4 pipérazinyl-1]-2 aza-1 spiro[4.5] décatétraène-1,3,6,9 one-8 en solution dans 150 $cm^3$ d'acide acétique et 80 $cm^3$ d'une solution d'acide bromhydrique à 48%. Après refroidissement à une température voisine de 20 °C, la solution est évaporée à sec sous pression réduite (20 mm de mercure; 2,7 kPa) à 40 °C. On ajoute au résidu 200 g de glace pillée puis 110 $cm^3$ d'une solution aqueuse d'ammoniaque 11N. La solution aqueuse est extraite 4 fois avec 400 $cm^3$ au total de chloroforme. Les fractions organiques sont réunies, lavées avec 50 $cm^3$ d'eau distillée, séchées sur sulfate de magnésium anhydre, filtrées et évaporées à sec sous pression réduite (20 mm de mercure; 2,7 kPa) à 40 °C. Le résidu obtenu est dissous dans 200 $cm^3$ de chlorure de méthylène et la solution est versée sur 120 g de silice contenus dans une colonne de 3,2 cm de diamètre. On élue d'abord avec 1,6 l de chlorure de méthylène et l'éluat correspondant est éliminé. On élue ensuite avec 1,4 l d'un mélange de chlorure de méthylène et de méthanol (95–5 en volumes) et l'éluat correspondant est évaporé à sec sous pression réduite (20 mm de mercure; 2,7 kPa) à 40 °C. On obtient ainsi 2,8 g d'(hydroxy-4 phényl)-4 [(méthyl-4 benzyl)-4 pipérazinyl-1]-2 aza-1 spiro[4.5] décatétraène-1,3,6,9 one-8 sous forme d'un solide cristallisé fondant à 140 °C.

Le (méthoxy-4 phényl)-1 [(méthyl-4 benzyl)-4 pipérazinyl-1]-2 aza-1 spiro[4.5] décatétraène-1,3,6,9 one-8 peut être préparé comme décrit à l'exemple 17.

Exemple 19

On chauffe pendant 4 h à une température voisine de 210 °C une solution de 2,8 g de (méthoxy-2 phényl)-4 [(méthyl-4 benzyl)-2 pipérazinyl-1]-2 aza-1 spiro[4.5] décatétraène-1,3,6,9 one-8 dans 80 $cm^3$ de trichloro-1,2,4 benzène. Après évaporation à sec sous pression réduite (20 mm de mercure; 0,13 kPa) à 40 °C, le résidu obtenu est dissous dans 50 $cm^3$ de chlorure de méthylène et la solution est versée sur 60 g de silice contenus dans une colonne de 2 cm de diamètre. On élue d'abord avec 200 $cm^3$ d'un mélange de chlorure de méthylène et de méthanol (99–1 en volumes); les éluats correspondants sont éliminés. On élue ensuite avec 450 $cm^3$ d'un mélange de chlorure de méthylène et de méthanol (99–1 en volumes) et l'éluat correspondant est évaporé à sec sous pression réduite (20 mm de mercure; 2,7 kPa) à 40 °C. Après recristallisation du résidu dans 75 $cm^3$ d'acétonitrile, on obtient 1,8 g de (méthoxy-2 phényl)-1 [(méthyl-4 benzyl)-4 pipérazinyl-1]-3 7H-pyrrolo[1,2-a] azépinone-7 fondant à 186 °C.

La (méthoxy-2 phényl)-4 [(méthyl-4 benzyl)-2 pipérazinyl-1]-2 aza-1 spiro[4.5] décatétraène-1,3,6,9 one-8 peut être préparée en opérant comme à l'exemple 11 mais à partir d'une solution agitée de 4,6 g de (méthoxy-2 phényl)-1 (méthoxy-4 phénylimino)-3 [(méthyl-4 benzyl)-4 pipérazinyl-1]3 propyne-1 et de 15 $cm^3$ d'acide acétique dans laquelle ont été ajoutés 25 $cm^3$ d'acide sulfurique. On laisse agiter pendant 30 min à température ambiante. Après trituration dans l'éther isopropylique, on obtient 2,8 g de [(méthyl-4 benzyl)-4 pipérazinyl-1]-2 (methoxy-2 phényl)-4 aza-1 spiro[4.5] décatétraène-1,3,6,9 one-8 fondant à 147 °C.

Le (méthoxy-2 phényl)-1 (méthoxy-4 phénylimino)-3 [(méthyl-4 benzyl)-4 pipérazinyl-1]-3 propyne-1 peut être préparé en opérant comme à l'exemple 11 mais à partir d'une solution de 20,6 g de N-(dichlorométhylène) p-anisidine dans 200 $cm^3$ d'éther éthylique à laquelle on ajoute une solution de 38,4 g de (méthyl-4 benzyl)-4 pipérazine dans 150 $cm^3$ de tétrahydrofuranne. La solution obtenue après filtration est ajoutée en 30 min

à une température voisine de –70 °C à une solution de (méthoxy-2 phényl)-2 ethynyllithium obtenue en ajoutant en 20 min, à une température voisine de –70 °C, 126 $cm^3$ d'une solution 1,6M de n-butyllithium dans l'hexane à une solution de 29,5 g de méthoxy-2 β,β-dibromostyrène dans 300 $cm^3$ de tétrahydrofuranne. On obtient ainsi 30,6 g de (méthoxy-2 phényl)-1 (méthoxy-4 phénylimino)-3 [(méthyl-4 benzyl)-4 pipérazinyl-1]-3 propyne-1 fondant à 83 °C.

Exemple 20

On chauffe pendant 11 h à une température voisine de 210 °C, une solution de 12,4 g d'[(éthoxycarbonyl-4 benzyl)-4 pipérazinyl-1]-2 phényl-4 aza-1 spiro[4.5] décatétraène-1,3,6,9 one-8 dans 250 $cm^3$ de trichloro-1,2,4 benzène. Après évaporation à sec sous pression réduite (1 mm de mercure; 0,13 kPa) à 40 °C, le résidu obtenu est dissous dans 250 $cm^3$ de chlorure de méthylène et la solution est versée sur 400 g de silice contenus dans une colonne de 6 cm de diamètre. On élue d'abord avec 2 l de chlorure de méthylène et les éluats correspondants sont éliminés. On élue ensuite avec 5 l d'une mélange de chlorure de méthylène et de méthanol (99–1 en volumes) et l'éluat correspondant est évaporé à sec sous pression réduite (20 mm de mercure; 2,7 kPa) à 40 °C. Après recristallisation du résidu dans 120 $cm^3$ d'éthanol, on obtient 5,7 g d'[(éthoxycarbonyl-4 benzyl)-4 pipérazinyl-1]-3 phényl-1 7H-pyrrolo[1,2-a] azépinone-7 fondant à 129 °C.

L'[(éthoxycarbonyl-4 benzyl)-4 pipérazinyl-1]-2 phényl-4 aza-1 spiro[4.5] décatétraène-1,3,6,9 one-8 peut être préparé en opérant comme à l'exemple 11 mais à partir de 135 $cm^3$ d'acide sulfurique et d'une solution agitée de 21,4 g d'[(éthoxycarbonyl-4 benzyl)-4 pipérazinyl-1]-3 (méthoxy-4 phénylimino)-3 phényl-1 propyné-1 dans 80 $cm^3$ d'acide acétique. Après trituration dans l'éther isopropylique, on obtient 12,4 g d'[(éthoxycarbonyl-4 benzyl)-4 pipérazinyl-1]-2 phényl-4 aza-1 spiro[4.5] décatétraène-1,3,6,9 one-8 fondant à 199 °C.

L'[(éthoxycarbonyl-4 benzyl)-4 pipérazinyl-1]-3 (méthoxy-4 phénylimino)-3 phényl-1 propyne-1 peut être préparé de la manière suivante: A une solution de 31,4 g de N-(dichlorométhylène) p-anisidine dans 200 $cm^3$ de tétrahydrofuranne sous atmosphère d'azote, on ajoute à une température voisine de 15 °C et en 15 min une solution de 38,2 g d'éthoxycarbonyl-4 benzyl-pipérazine dans 250 $cm^3$ de tétrahydrofuranne et poursuit l'agitation pendant encore 1 h à une température voisine de 20 °C. Le précipité qui s'est formé est séparé par filtration. La solution ainsi obtenue est ajoutée en 30 min à une température voisine de –70 °C à une solution de phényléthynyllithium dans 200 $cm^3$ de tétrahydrofuranne obtenue en faisant réagir à une température voisine de –70 °C 64,1 g de phénylacétylène dissous dans 200 $cm^3$ de tétrahydrofuranne avec 385 $cm^3$ d'une solution 1,6M de n-butyllithium dans l'hexane. On laisse réagir pendant 1 h et 30 min à –70 °C, puis pendant 2 h à –20 °C et on laisse ensuite réchauffer à une température voisine de 20 °C. On évapore à sec sous pression réduite (20 mm de mercure; 2,7 kPa) à 40°C, le résidu obtenu est repris dans 700 $cm^3$ d'eau distillée et extrait avec 4 fois 500 $cm^3$ de chlorure de méthylène. Les fractions orgniques sont réunies et lavées avec 500 $cm^3$ d'eau distillée, séchées sur sulfate de magnésium anhydre, filtrées puis évaporées à sec sous pression réduite (20 mm de mercure; 2,7 kPa) à 40 °C. Le résidu obtenu est dissous dans 200 $cm^3$ de chlorure de méthylène et la solution est versée sur 2 kg de silice contenus dans une colonne de 8 cm de diamètre. On élue d'abord avec 1 l de chlorure de méthylène puis avec 1 l d'un mélange de chlorure de méthylène et de méthanol (99–1 en volumes); les éluats correspondants sont éliminés. On élue ensuite avec 15 l d'un mélange de chlorure de méthylène et de méthanol (99–1 en volumes) et l'éluat correspondant est évaporé à sec sous pression réduite (20 mm de mercure; 2,7 kPa) à 40 °C. On obtient ainsi 21,4 g d'[(éthoxycarbonyl-4 benzyl)-4 pipérazinyl-1]-3 (méthoxy-4 phénylimino)-3 phényl-1 propyne-1, sous forme d'une huile brune.

Rf = 0,36 [chromatographie sur couche mince de gel de silice; éluant: chlorure de méthylène-méthanol (96–4 en volumes)].

Exemple 21

A une suspension agitée sous atmosphère d'azote de 1,2 g d'[(éthoxycarbonyl-4 benzyl)-4 pipérazinyl-1]-3 phényl-1 7H-pyrrolo[1,2-a] azépinone-7 préparé comme décrit à l'exemple 20 et de 30 $cm^3$ d'alcool éthylique, on ajoute à une température voisine de 20 °C et pendant 5 min une solution de 0,2 g d'hydroxyde de potassium dans 3 $cm^3$ d'eau distillée. Le mélange réactionnel est ensuite chauffé à 60 °C pendant 4 h. Après refroidissement de la solution, on ajoute 10 $cm^3$ d'une solution aqueuse d'hydroxyde de potassium 1N, et on lave avec 3 fois 150 $cm^3$ au total de chlorure de méthylène. Les phases organiques sont réunies, séchées sur sulfate de magnésium anhydre, filtrées et évaporées à sec sous pression réduit (20 mm de mercure; 2,7 kPa) à 40 °C. Le résidu est dissous dans 5 $cm^3$ de chlorure de méthylène et la solution est versée sur 40 g de silice contenus dans une colonne de 1 cm de diamètre. On élue d'abord avec 2,5 l d'un mélange de cyclohexane et d'acétate d'éthyle (50–50 en volumes) et l'éluat correspondant est éliminé. On élue ensuite avec 1 l de méthanol et l'éluat correspondant est évaporé à sec sous pression réduite (20 mm de mercure; 2,7 kPa) à 40 °C. Après recristallisation dans le méthyl-2 butanol-2, on obtient 0,6 g de [(carboxy-4 benzyl)-4 pipérazinyl-1]-3 phényl-1 7H-pyrrolo[1,2-a] azépinone-7 sous forme de son sel de potassium.

Rf = 0,30 [chromatographie sur couche mince de silice; éluant: chloroforme-méthanol (98–2 en volumes)].

La présente invention concerne également les médicaments constitués par un produit de formule générale (I), sous forme libre ou sous forme de sel d'addition avec un acide ou une base pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale ou rectale.

Comme compositions solides pour administration orale peuvent être utilisés des comprimés, pilules, poudres (notamment dans des capsules de gélatine ou des cachets) ou granulés. Dans ces compositions, le produit actif selon l'invention est mélangé à une ou plusieurs diluants inertes, tels que amidon, cellulose, saccharose, lactose ou silice. Ces compositions peuvent également comprendre des substances autres que les diluants, par exemple un ou plusieurs lubrifiants tel que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent également comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

En thérapeutique humaine, les produits selon l'invention sont particulièrement utiles dans le traitement des troubles du psychisme et plus particulièrement des psychoses telles que la schizophrénie ou les délires. Les doses dépendent de l'effet recherché et de la durée du traitement; elles sont généralement comprises entre 25 et 250 mg par jour par voie orale pour un adulte en une ou plusieurs prises.

D'une façon générale, le médecin déterminera la posologie qu'il estime la plus appropriée en fonction de l'âge, du poids et de tous les autres facteurs propres au sujet à traiter.

Les exemples suivants, donnés à titre non limitatif, illustrent des compositions selon l'invention.

Exemple A

On prépare, selon la technique habituelle, des comprimés dosés à 25 mg de produit actif ayant la composition suivante:

| | |
|---|---|
| – [(méthyl-4 benzyl)-4 pipérazinyl-1]-3 phényl-1 7H-pyrrolo[1,2-a] azépinone-7 | 25 mg |
| – amidon | 60 mg |
| – lactose | 50 mg |
| – stéarate de magnésium | 2 mg |

Exemple B

On prépare une solution injectable contenant 25 mg de produit actif ayant la composition suivante:

| | |
|---|---|
| – [(méthyl-4 benzyl)-4 pipérazinyl-1]-3 phényl-1 7H-pyrrolo[1,2-a] azépinone-7 | 25 mg |
| – solution aqueuse d'acide méthanesulfonique 0,1N | 1,23 $cm^3$ |
| – soluté injectable qsp | 12,5 $cm^3$ |

**Revendications pour les Etats contractants: BE, CH, DE, FR, GB, IT, LI, LU, NL, SE**

1. Nouveau dérivé de la pyrrolo[1,2-a]-azépinone, caractérisé en ce qu'il répond à la formule générale:

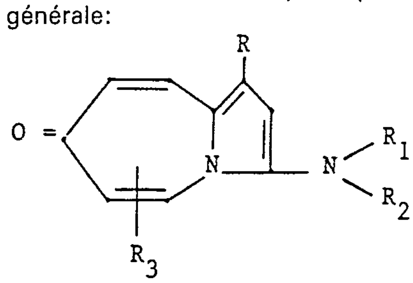

(I)

dans laquelle $R_3$ représente un atome d'hydrogène ou d'halogène et

A) soit R représente un radical phénylthio et $R_1$ et $R_2$ forment ensemble avec l'atome d'azote auquel ils sont liés un cycle pipérazine substitué sur le second atome d'azote par un radical alcoyle, alcényle contenant 2 à 4 atomes de carbone,

B) soit R représente un radical phényle éventuellement substitué par un ou plusieurs atomes d'halogène ou radicaux hydroxy, alcoyle, alcoyloxy ou alcoylthio et $R_1$ et $R_2$ forment ensemble avec l'atome d'azote auquel ils sont liés un cycle pipérazine ou homopipérazine substitués sur le second atome d'azote par un radical hydroxyalcoyle dont la partie alcoyle contient 2 à 4 atomes de carbone, alcényle contenant 2 à 4 atomes de carbone, alcynyle contenant 2 à 4 atomes de carbone, benzyle ou phénéthyle dont les parties phényle peuvent être éventuellement substituées par un atome d'halogène ou un radical alcoyle, alcoyloxy, alcoylthio, cyano, trifluorométhyle, carboxy, carboxyalcoyle, alcoyloxycarbonyle, alcoyloxycarbonylalcoyle ou hydroxyalcoyle étant entendu que les radicaux alcoyle et portions alcoyle citées

dans la définition ci-dessus contiennent, sauf mention spéciale, 1 à 4 atomes de carbone en chaîne droite ou ramifiée, ainsi que ses sels pharmaceutiquement acceptables.

2. Procédé de préparation d'un produit selon la revendication 1 dans la formule duquel les symboles sont définis comme à la revendication 1 à l'exception d'un produit dans la formule duquel $R_1$ et $R_2$ forment ensemble avec l'atome d'azote auquel ils sont liés un cycle pipérazine ou homopipérazine substitués sur le second atome d'azote par un radical hydroxyalcoyle dont la partie alcoyle contient 2 à 4 atomes de carbone, alcynyle contenant 2 à 4 atomes de carbone, benzyle ou phénéthyle substitués par un radical cyano, caractérisé en ce que l'on procède à la transposition d'un produit de formule générale:

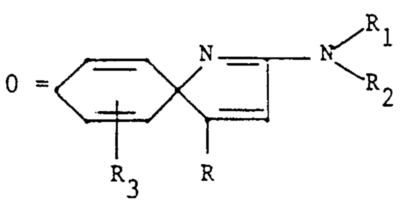

(II)

dans laquelle R et $R_3$ sont définis comme à la revendication 1 et $R_1$ et $R_2$ sont définis comme à la revendication 1 à l'exception de former avec l'atome d'azote auquel ils sont liés un cycle pipérazine ou homopipérazine substitués sur le second atome d'azote par un radical hydroxyalcoyle dont la partie alcoyle contient 2 à 4 atomes de carbone, alcynyle contenant 2 à 4 atomes de carbone, benzyle ou phénéthyle substitués par un radical cyano, carboxy, carboxyalcoyle ou hydroxyalcoyle, pour obtenir un produit de formule générale (I) dont les symboles sont définis comme à la revendication 1 à l'exception pour $R_1$ et $R_2$ de former ensemble avec l'atome d'azote auquel ils sont liés un cycle pipérazine ou homopipérazine substitués sur le second atome d'azote par un radical hydroxyalcoyle dont la partie alcoyle contient 2 à 4 atomes de carbone, alcynyle contenant 2 à 4 atomes de carbone, benzyle ou phénéthyle substitués par un radical cyano, carboxy, carboxyalcoyle ou hydroxyalcoyle, isole le produit obtenu, puis, si on désire obtenir un produit de formule générale (I) dans laquelle R et $R_3$ sont définis comme à la revendication 1 et $R_1$ et $R_2$ forment avec l'atome d'azote auquel ils sont liés un cycle pipérazine ou homopipérazine substitués sur le second atome d'azote par un radical benzyle ou phénéthyle dont les parties phényle sont substituées par un radical carboxy, carboxyalcoyle on transforme le produit correspondant de formule générale (I) dans laquelle R et $R_3$ sont définis comme à la revendication 1 et $R_1$ et $R_2$ forment avec l'atome d'azote auquel ils sont liés un cycle pipérazine ou homopipérazine substitués sur le second atome d'azote par un radical benzyle ou phénéthyle dont les portions phényle sont substituées par un radical alcoyloxycarbonyle ou alcoyloxycarbonylalcoyle, par toute méthode connue pour transformer un ester en acide ou en alcool sans toucher au reste de la molécule, puis isole le produit obtenu et le transforme éventuellement en un sel pharmaceutiquement acceptable.

3. Procédé de préparation d'un produit selon la revendication 1 dans la formule duquel $R_3$ est défini comme à la revendication 1 et les autres symboles sont définis comme à la revendication 1 en B), caractérisé en ce que l'on fait réagir un produit de formule générale:

$$R_4X \qquad \text{(VIII)}$$

dans laquelle $R_4$ représente un radical hydroxyalcoyle dont la partie alcoyle contient 2 à 4 atomes de carbone, alcényle contenant 2 à 4 atomes de carbone, alcynyle contenant 2 à 4 atomes de carbone, benzyle ou phénéthyle dont les portions phényle peuvent être éventuellement substituées par un atme d'halogène ou un radical alcoyle, alcoyloxy, alcoylthio, cyano, trifluorométhyle, carboxy, carboxyalcoyle, alcoyloxycarbonyle, alcoyloxycarbonylalcoyle ou hydroxyalcoyle et X représente un atome d'halogène, sur un produit de formule générale:

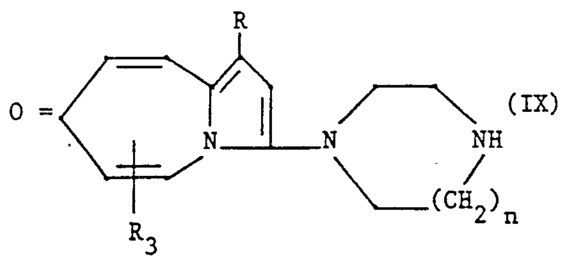

(IX)

dans laquelle n est égal à 1 ou 2, $R_2$ est défini comme à la revendication 1 et R est défini comme à la revendication 1 en B), puis isole le produit obtenu et le transforme éventuellement en un sel pharmaceutiquement acceptable.

4. Composition pharmaceutique, caractérisée en ce qu'elle contient au moins un dérivé selon la revendication 1 en association avec un ou plusieurs diluants ou adjuvants compatibles et pharmaceutiquement acceptables.

**Revendication pour l'Etat contractant: AT**

1. Procédé de préparation d'un nouveau dérivé de la pyrrolo-[1,2-a] azépinone de formule générale:

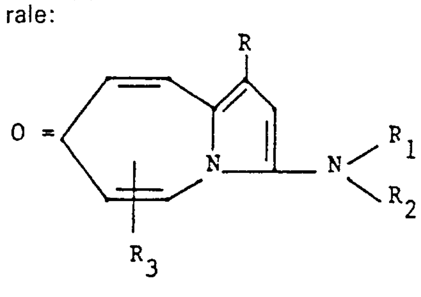

(I)

dans laquelle $R_3$ représente un atome d'hydrogène ou d'halogène et

A) soit R représente un radical phénylthio et $R_1$ et $R_2$ forment ensemble avec l'atome d'azote auquel ils sont liés un cycle pipérazine substitué sur le second atome d'azote par un radical alcoyle, alcényle contenant 2 à 4 atomes de carbone,

B) soit R représente un radical phényle éventuellement substitué par un ou plusieurs atomes d'halogène ou radicaux hydroxy, alcoyle, alcoyloxy ou alcoylthio et $R_1$ et $R_2$ forment ensemble avec l'atome d'azote auquel ils sont liés un cycle pipérazine ou homopipérazine substitués sur le second atome d'azote par un radical hydroxyalcoyle dont la partie alcoyle contient 2 à 4 atomes de carbone, alcényle contenant 2 à 4 atomes de carbone, alcynyle contenant 2 à 4 atomes de carbone, benzyle ou phénéthyle dont les parties phényle peuvent être éventuellement substituées par un atome d'halogène ou un radical alcoyle, alcoyloxy, alcoylthio, cyano, trifluorométhyle, carboxy, carboxyalcoyle, alcoyloxycarbonyle, alcoyloxycarbonylalcoyle ou hydroxyalcoyle, étant entendu que les radicaux alcoyle et portions alcoyle citées dans la définition ci-dessus contiennent, sauf mention spéciale, 1 à 4 atomes de carbone en chaîne droite ou ramifiée, ainsi que ses sels pharmaceutiquement acceptables, caractérisé en ce que,

– pour la préparation d'un produit de formule générale (I) dans laquelle les symboles sont définis comme précédemment à l'exception d'un produit dans la formule duquel $R_1$ et $R_2$ forment ensemble avec l'atome d'azote auquel ils sont liés un cycle pipérazine ou homopipérazine substitués sur le second atome d'azote par un radical hydroxyalcoyle dont la partie alcoyle contient 2 à 4 atomes de carbone, alcynyle contenant 2 à 4 atomes de carbone, benzyle ou phénéthyle substitués par un radical cyano, on procède à la transposition d'un produit de formule générale:

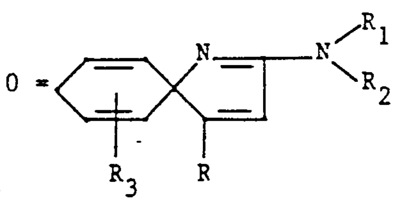
(II)

dans laquelle R et $R_3$ sont définis comme précédemment et $R_1$ et $R_2$ sont définis comme précédemment à l'exception de former avec l'atome d'azote auquel ils sont liés un cycle pipérazine ou homopipérazine substitués sur le second atome d'azote par un radical hydroxyalcoyle dont la partie alcoyle contient 2 à 4 atomes de carbone, alcynyle contenant 2 à 4 atomes de carbone, benzyle ou phénéthyle substitués par un radical cyano, carboxy, carboxyalcoyle ou hydroxyalcoyle, pour obtenir un produit de formule générale (I) dont les symboles sont définis comme précédemment à l'exception pour $R_1$ et $R_2$ de former ensemble avec l'atome d'azote auquel ils sont liés un cycle pipérazine ou homopipérazine substitués sur le second atome d'azote par un radical hydroxyalcoyle dont la partie alcoyle contient 2 à 4 atomes de carbone, alcynyle contenant 2 à 4 atomes de carbone, benzyle ou phénéthyle substitués par un radical cyano, carboxy, carboxyalcoyle ou hydroxyalcoyle isole le produit obtenu, puis, si on désire obtenir un produit de formule générale (I) dans laquelle R et $R_3$ sont définis comme précédemment et $R_1$ et $R_2$ forment avec l'atome d'azote auquel ils sont liés un cycle pipérazine ou homopipérazine substitués sur le second atome d'azote par un radical benzyle ou phénéthyle dont les parties phényle sont substituées par un radical carboxy, carboxyalcoyle on transforme le produit correspondant de formule générale (I) dans laquelle R et $R_3$ sont définis comme précédemment et $R_1$ et $R_2$ forment avec l'atome d'azote auquel ils sont liés un cycle pipérazine ou homopipérazine substitués sur le second atome d'azote par un radical benzyle ou phénéthyle dont les portions phényle sont substituées par un radical alcoyloxycarbonyle ou alcoyloxycarbonylalcoyle, par toute méthode connue pour transformer un ester en acide ou en alcool sans toucher au reste de la molécule, puis isole le produit obtenu et le transforme éventuellement en un sel pharmaceutiquement acceptable, ou en ce que,

– pour la préparation d'un produit de formule générale (I) dans laquelle $R_3$ est défini comme précédemment et les autres symboles sont définis comme précédemment en B), on fait réagir un produit de formule générale:

$$R_4X \qquad (VIII)$$

dans laquelle $R_4$ représente un radical hydroxyalcoyle dont la partie alcoyle contient 2 à 4 atomes de carbone, alcényle contenant 2 à 4 atomes de carbone, alcynyle contenant 2 à 4 atomes de carbone, benzyle ou phénéthyle dont les portions phényle peuvent être éventuellement substituées par un atome d'halogène ou un radical alcoyle, alcoyloxy, alcoylthio, cyano, trifluorométhyle, carboxy, carboxyalcoyle, alcoyloxycarbonyle, alcoyloxycarbonylalcoyle ou hydroxyalcoyle et X représente un atome d'halogène, sur un produit de formule générale:

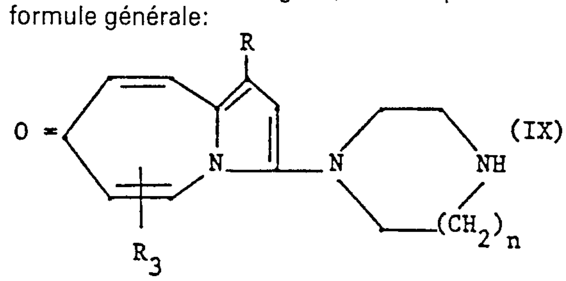
(IX)

dans laquelle n est égal à 1 ou 2, $R_3$ est défini comme précédemment et R est défini comme précédemment en B), puis isole le produit obtenu et le transforme éventuellement en un sel pharmaceutiquement acceptable.

**Patentansprüche für die Vertragsstaaten BE, CH, DE, FR, GB, IT, LI, LU, NL, SE**

1. Neues Derivat des Pyrrolo[1,2-a]azepinons, dadurch gekennzeichnet, daß es der allgemeinen

Formel:

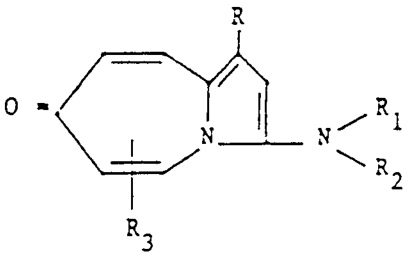

(I)

entspricht, worin $R_3$ ein Wasserstoff- oder Halogenatom bedeutet und

A) entweder R einen Phenylthiorest bedeutet und $R_1$ und $R_2$ zusammen mit dem Stickstoffatom, an das sie gebunden sind, einen Piperazinring bilden, der an dem zweiten Stickstoffatom durch einen Alkyl-, Alkenylrest mit 2 bis 4 Kohlenstoffatomen substituiert ist, oder

B) R bedeutet einen Phenylrest, der gegebenenfalls durch einen oder mehrere Halogenatome oder Hydroxy-, Alkyl-, Alkyloxy- oder Alkylthioreste substituiert ist und $R_1$ und $R_2$ bilden zusammen mit dem Stickstoffatom, an das sie gebunden sind, einen Piperazin- oder Homopiperazinring, die am zweiten Stickstoffatom substituiert sind durch einen Hydroxyalkylrest, dessen Alkylteil 2 bis 4 Kohlenstoffatome enthält, Alkenyl, enthaltend 2 bis 4 Kohlenstoffatome, Alkinyl, enthaltend 2 bis 4 Kohlenstoffatome, Benzyl oder Phenethyl, deren Phenylteile gegebenenfalls substituiert sein können durch ein Halogenatom oder einen Alkyl-, Alkyloxy-, Alkylthio-, Cyano-, Trifluormethyl-, Carboxy-, Carboxyalkyl-, Alkyloxycarbonyl-, Alkyloxycarbonylalkyl- oder Hydroxyalkylrest, wobei die in der vorstehenden Definition angegebenen Alkylreste und Alkylteile, wenn nichts anderes angegeben ist, 1 bis 4 Kohlenstoffatome in gerader oder verzweigter Kette enthalten, sowie seine pharmazeutisch annehmbaren Salze.

2. Verfahren zur Herstellung eines Produktes gemäß Anspruch 1, in dessen Formel die Symbole wie in Anspruch 1 definiert sind, mit Ausnahme eines Produkts, in dessen Formel $R_1$ und $R_2$ zusammen mit dem Stickstoffatom, an das sie gebunden sind, einen Piperazin- oder Homopiperazinring bilden, der am zweiten Stickstoffatom substituiert ist durch einen Hydroxyalkylrest, dessen Alkylteil 2 bis 4 Kohlenstoffatome enthält, Alkinyl, enthaltend 2 bis 4 Kohlenstoffatome, Benzyl oder Phenethyl, die durch einen Cyanorest substituiert sind, dadurch gekennzeichnet, daß man die Umlagerung eines Produkts der allgemeinen Formel:

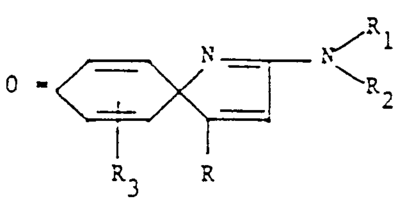

(II)

durchführt, worin R und $R_3$ wie in Anspruch 1 definiert sind und $R_1$ und $R_2$ wie in Anspruch 1 definiert sind, mit der Ausnahme, daß sie mit dem Stickstoffatom, an das sie gebunden sind, einen Piperazin- oder Homopiperazinring bilden, die an dem zweiten Stickstoffatom substituiert sind durch einen Hydroxyalkylrest, dessen Alkylteil 2 bis 4 Kohlenstoffatome enthält, Alkinyl, enthaltend 2 bis 4 Kohlenstoffatome, Benzyl oder Phenethyl, substituiert durch einen Cyano-, Carboxy-, Carboxyalkyl- oder Hydroxyalkylrest, um ein Produkt der allgemeinen Formel (I) zu erhalten, deren Symbole wie in Anspruch 1 definiert sind, mit der Ausnahme, daß $R_1$ und $R_2$ zusammen mit dem Stickstoffatom, an das sie gebunden sind, einen Piperazin- oder Homopiperazinring bilden, der am zweiten Stickstoffatom substituiert ist durch einen Hydroxyalkylrest, dessen Alkylteil 2 bis 4 Kohlenstoffatome enthält, Alkinyl, enthaltend 2 bis 4 Kohlenstoffatome, Benzyl oder Phenethyl, substituiert durch einen Cyano-, Carboxy-, Carboxyalkyl- oder Hydroxyalkylrest, daß man das erhaltene Produkt isoliert und dann, falls man ein Produkt der allgemeinen Formel (I) zu erhalten wünscht, worin R und $R_3$ wie in Anspruch 1 definiert sind und $R_1$ und $R_2$ mit dem Stickstoffatom, an das sie gebunden sind, einen Piperazin- oder Homopiperazinring bilden, der am zweiten Stickstoffatom substituiert ist durch einen Benzyl- oder Phenethylrest, dessen Phenylteile substituiert sind durch einen Carboxy-, Carboxyalkylrest, man das entsprechende Produkt der allgemeinen Formel (I), worin R und $R_3$ wie in Anspruch 1 definiert sind und $R_1$ und $R_2$ zusammen mit dem Stickstoffatom, an das sie gebunden sind, einen Piperazin- oder Homopiperazinring bilden, der am zweiten Stickstoffatom durch einen Benzyl- oder Phenethylrest substituiert ist, deren Phenylteile durch einen Alkoxycarbonyl- oder Alkyloxycarbonylalkylrest substituiert sind, durch jede Methode, die zur Umwandlung eines Esters in eine Säure oder einen Alkohol bekannt ist, ohne daß der Rest des Moleküls berührt wird, umwandelt, das erhaltene Produkt dann isoliert und es gegebenenfalls in ein pharmazeutisch annehmbares Salz überführt.

3. Verfahren zur Herstellung eines Produkts gemäß Anspruch 1, in dessen Formel $R_3$ wie in Anspruch 1 definiert ist und die übrigen Symbole wie in Anspruch 1 unter B) definiert sind, dadurch gekennzeichnet, daß man ein Produkt der allgemeinen Formel:

$$R_4X \quad \text{(VIII)}$$

worin $R_4$ bedeutet einen Hydroxyalkylrest, dessen Alkylteil 2 bis 4 Kohlenstoffatome enthält, Alkenyl, enthaltend 2 bis 4 Kohlenstoffatome, Alkinyl, enthaltend 2 bis 4 Kohlenstoffatome, Benzyl oder Phenethyl, dessen Phenylteile gegebenenfalls substituiert sein können durch ein Halogenatom oder einen Alkyl-, Alkyloxy-, Alkylthio-, Cyano-, Trifluormethyl-, Carboxy-, Carboxyalkyl-, Alkyloxycarbonyl-, Alkyloxycarbonylalkyl- oder Hydroxyalkylrest und X ein Halogenatom bedeutet, auf

ein Produkt der allgemeinen Formel:

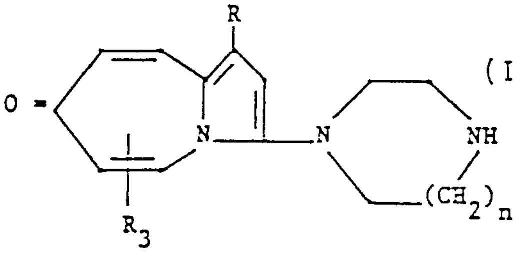

(IX)

einwirken läßt, worin n gleich 1 oder 2 ist, $R_3$ wie in Anspruch 1 definiert ist und R wie in Anspruch 1 unter B) definiert ist, das erhaltene Produkt dann isoliert und es gegebenenfalls in ein pharmazeutisch annehmbares Salz überführt.

4. Pharmazeutische Zusammensetzung, dadurch gekennzeichnet, daß sie wenigstens ein Derivat gemäß Anspruch 1 enthält, zusammen mit einem oder mehreren verträglichen und pharmazeutisch annehmbaren Verdünnungs- oder Hilfsmitteln.

**Patentanspruch für den Vertragsstaat AT**

1. Verfahren zur Herstellung eines neuen Pyrrolo-[1,2-a]-azepinon-derivats der allgemeinen Formel:

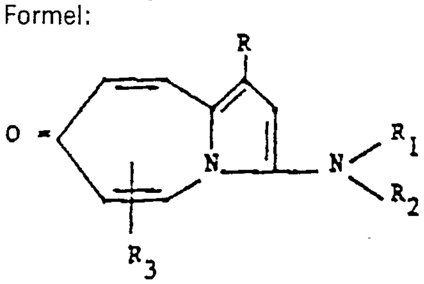

(I)

in welcher $R_3$ ein Wasserstoff oder Halogenatom darstellt und

A) entweder R einen Phenylthiorest darstellt und $R_1$ und $R_2$ zusammen mit dem Stickstoffatom, an das sie gebunden sind, einen Piperazinring, substituiert am zweiten Stickstoffatom durch einen Alkyl- oder Alkenylrest mit 2 bis 4 Kohlenstoffatomen, bilden,

B) oder R einen gegebenenfalls durch ein oder mehrere Halogenatome oder Hydroxy-, Alkyl-, Alkyloxy- oder Alkylthioreste substituierten Phenylrest darstellt und $R_1$ und $R_2$ zusammen mit dem Stickstoffatom, an das sie gebunden sind, einen Piperazin- oder Homopiperazinring bilden, der am zweiten Stickstoffatom substituiert ist durch einen Hydroxyalkylrest, dessen Alkylteil 2 bis 4 Kohlenstoffatome enthält, Alkenylrest mit 2 bis 4 Kohlenstoffatomen, Alkynylrest mit 2 bis 4 Kohlenstoffatomen, Benzyl- oder Phenäthylrest, dessen Phenylteile gegebenenfalls durch ein Halogenatom oder einen Alkyl-, Alkyloxy-, Alkylthio-, Cyano-, Trifluormethyl-, Carboxy-, Carboxyalkyl-, Alkyloxycarbonyl-, Alkyloxycarbonylalkyl- oder Hydroxyalkylrest substituiert sein können, wobei die in der obigen Definition genannten Alkylreste und -teile ohne speziellen Hinweis 1 bis 4 Kohlenstoffatome in gerader oder verzweigter Kette enthalten, sowie von seinen pharmazeutisch zulässigen Salzen, dadurch gekennzeichnet, daß man

– zur Herstellung einer Verbindung der allgemeinen Formel (I), in welcher die Symbole die obige Bedeutung haben, mit Ausnahme einer Verbindung, in deren Formel $R_1$ und $R_2$ zusammen mit dem Stickstoffatom, an das sie gebunden sind, einen Piperazin- oder Homopiperazinring bilden, der am zweiten Stickstoffatom substituiert ist durch einen Hydroxyalkylrest, dessen Alkylteil 2 bis 4 Kohlenstoffatome enthält, einen Alkynylrest mit 2 bis 4 Kohlenstoffatomen, einen Benzyl- oder Phenäthylrest, substituiert durch einen Cyanorest, die Umlagerung einer Verbindung der allgemeinen Formel:

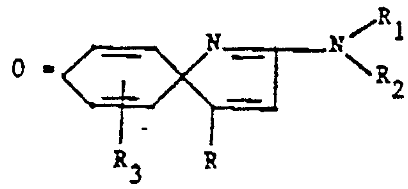

(II)

in welcher R und $R_3$ die obige Bedeutung haben und $R_1$ und $R_2$ die obige Bedeutung haben, ausgenommen, daß sie mit dem Stickstoffatom, an das sie gebunden sind, einen Piperazin- oder Homopiperazinring bilden, der am zweiten Stickstoffatom substituiert ist durch einen Hydroxyalkylrest, dessen Alkylteil 2 bis 4 Kohlenstoffatome enthält, einen Alkynylrest mit 2 bis 4 Kohlenstoffatomen, einen Benzyl oder Phenäthylrest, substituiert durch einen Cyano-, Carboxy-, Carboxyalkyl- oder Hydroxyalkylrest, durchführt unter Bildung einer Verbindung der allgemeinen Formel (I), in welcher die Symbole die obige Bedeutung haben mit der Ausnahme, daß $R_1$ und $R_2$ zusammen mit dem Stickstoffatom, an das sie gebunden sind, einen Piperazin- oder Homopiperazinring bilden, der am zweiten Stickstoffatom substituiert ist durch einen Hydroxyalkylrest, dessen Alkylteil 2 bis 4 Kohlenstoffatome enthält, einen Alkynylrest mit 2 bis 4 Kohlenstoffatomen, einen Benzyl- oder Phenäthylrest, substituiert durch einen Cyano-, Carboxy-, Carboxyalkyl- oder Hydroxyalkylrest, das erhaltene Produkt isoliert und dann, falls man eine Verbindung der allgemeinen Formel (I), in der R und $R_3$ die obige Bedeutung haben und $R_1$ und $R_2$ zusammen mit dem Stickstoffatom, an das sie gebunden sind, einen Piperazin- oder Homopiperazinring bilden, der am zweiten Stickstoffatom substituiert ist durch einen Benzyl- oder Phenäthylrest, dessen Phenylteile durch einen Carboxy- oder Carboxyalkylrest substituiert sind, herstellen will, die entsprechende Verbindung der allgemeinen Formel (I), in welcher R und $R_3$ die obige Bedeutung haben und $R_1$ und $R_2$ zusammen mit dem Stickstoffatom, an das sie gebunden sind, einen Piperazin- oder Homopiperazinring bilden, der am zweiten Stickstoffatom substituiert ist durch einen Benzyl- oder Phenäthylrest, dessen Phenylteile durch einen Alkyloxycarbonyl- oder Alkyloxycarbonylalkylrest substituiert sind, nach

irgendeiner bekannten Methode zur Umwandlung eines Esters in eine Säure oder einen Alkohol, ohne den Rest des Moleküls anzugreifen, umwandelt und dann die erhaltene Verbindung isoliert und sie gegebenenfalls in ein pharmazeutisch zulässiges Salz umwandelt, oder dass man

– zur Herstellung einer Verbindung der allgemeinen Formel (I), worin $R_3$ die obige Bedeutung hat und die anderen Symbole die zuvor unter B) angegebene Bedeutung haben, eine Verbindung der allgemeinen Formel:

$$R_4X \qquad (VIII),$$

in welcher $R_4$ einen Hydroxyalkylrest, dessen Alkylteil 2 bis 4 Kohlenstoffatome enthält, einen Alkenylrest mit 2 bis 4 Kohlenstoffatomen, einen Alkynylrest mit 2 bis 4 Kohlenstoffatomen, einen Benzyl- oder Phenäthylrest, dessen Phenylteile gegebenenfalls durch ein Halogenatom oder einen Alkyl-, Alkyloxy-, Alkylthio-, Cyano-, Trifluormethyl-, Carboxy-, Carboxyalkyl-, Alkyloxycarbonyl-, Alkyloxycarbonylalkyl- oder Hydroxyalkylrest substituiert sind, darstellt und X für ein Halogenatom steht, mit einer Verbindung der allgemeinen Formel:

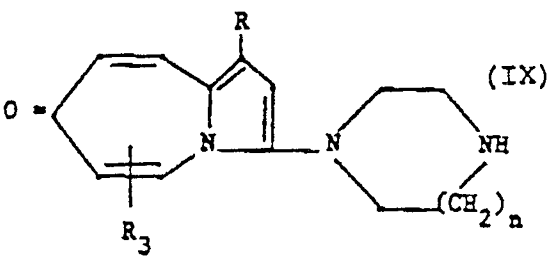

(IX)

in welcher n gleich 1 oder 2 ist, $R_3$ die obige Bedeutung hat und R die oben unter B) angegebene Bedeutung hat, umsetzt, dann die erhaltene Verbindung isoliert und sie gegebenenfalls in ein pharmazeutisch zulässiges Salz umwandelt.

**Claims for the contracting States BE, CH, DE, FR, GB, IT, LI, LU, NL, SE**

1. A new pyrrolo[1,2-a]azepinone derivative, which is of the general formula:

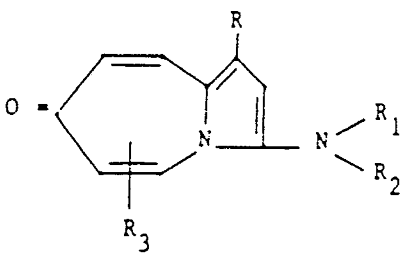

(I)

in which $R_3$ denotes a hydrogen or halogen atom, and

A) either R denotes a phenylthio radical and $R_1$ and $R_2$ form, together with the nitrogen atom to which they are linked, a piperazine ring substituted on the second nitrogen atom with an alkyl radical or alkenyl radical containing 2 to 4 carbon atoms,

B) or R denotes a phenyl radical optionally substituted with one or more halogen atoms or hydroxy, alkyl, alkyloxy or alkylthio radicals, and $R_1$ and $R_2$ form, together with the nitrogen atom to which they are linked, a piperazine or homopiperazine ring substituted on the second nitrogen atom with a hydroxyalkyl radical in which the alkyl portion contains 2 to 4 carbon atoms, an alkenyl radical containing 2 to 4 carbon atoms, an alkynyl radical containing 2 to 4 carbon atoms, or a benzyl or phenethyl radical in which the phenyl portions can optionally be substituted with a halogen atom or an alkyl, alkyloxy, alkylthio, cyano, trifluoromethyl, carboxy, carboxyalkyl, alkyloxycarbonyl, alkyloxycarbonylalkyl or hydroxyalkyl radical, with the understanding that the alkyl radicals and alkyl portions mentioned in the definition above contain, except where otherwise stated, 1 to 4 carbon atoms in a straight or branched chain, as well as the pharmaceutically acceptable salts thereof.

2. A process for preparing a product according to claim 1 in the formula of which the symbols are defined as in claim 1, with the exception of a product in the formula of which $R_1$ and $R_2$ form, together with the nitrogen atom to which they are linked, a piperazine or homopiperazine ring substituted on the second nitrogen atom with a hydroxyalkyl radical in which the alkyl portion contains 2 to 4 carbon atoms, an alkynyl radical containing 2 to 4 carbon atoms, or a benzyl or phenethyl radical substituted with a cyano radical, wherein the rearrangement is carried out of a product of general formula:

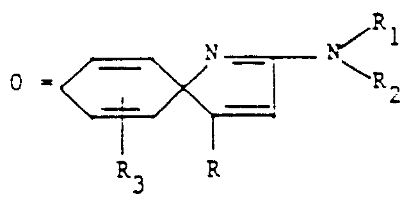

(II)

in which R and $R_3$ are defined as in claim 1, and $R_1$ and $R_2$ are defined as in claim 1 with the exception of forming, with the nitrogen atom to which they are linked, a piperazine or homopiperazine ring substituted on the second nitrogen atom with a hydroxyalkyl radical in which the alkyl portion contains 2 to 4 carbon atoms, an alkynyl radical containing 2 to 4 carbon atoms, or a benzyl or phenethyl radical substituted with a cyano, carboxy, carboxyalkyl or hydroxyalkyl radical, to obtain a product of general formula (I) in which the symbols are defined as in claim 1, with the exception or $R_1$ and $R_2$ forming, together with the nitrogen atom to which they are linked, a piperazine or homopiperazine ring substituted on the second nitrogen atom with a hydroxyalkyl radical in which the alkyl portion contains 2 to 4 carbon atoms, an alkynyl radical containing 2 to 4 carbon atoms, or a benzyl or phenethyl radical substituted with a cyano, carboxy, carboxyalkyl or hydroxyalkyl radical, the product obtained is isolated and then, if it is desired to obtain a product of general formula (I) in which R and $R_3$ are defined as in claim 1 and $R_1$ and $R_2$ form, with the nitrogen atom to which they are linked, a piperazine or homopiperazine ring substituted on the second nitrogen atom with a benzyl radical or a phenethyl radical in which the phenyl portions are substituted with a carboxy or carboxyalkyl radical, the corresponding product of general formula (I), in which R and $R_3$ are defined as in claim 1 and $R_1$ and $R_2$ form, with the nitrogen atom to which they are linked, a piperazine or homopiperazine ring substituted on the second nitrogen atom with a benzyl or phenethyl radical in which the phenyl portions are substituted with an alkyloxycarbonyl or alkyloxycarbonylalkyl radical, is converted by any known method for converting an ester to an acid or alcohol without affecting the remainder of the molecule, and the product obtained is then isolated and optionally converted to a pharmaceutically acceptable salt.

3. A process for preparing a product according to claim 1 in the formula of which $R_3$ is defined as in claim 1 and the other symbols are defined as in claim 1 at B), wherein a product of general formula:

$$R_4X \qquad (VIII)$$

in which $R_4$ denotes a hydroxyalkyl radical in which the alkyl portion contains 2 to 4 carbon atoms, an alkenyl radical containing 2 to 4 carbon atoms, an alkynyl radical containing 2 to 4 carbon atoms, or a benzyl or phenethyl radical in which the phenyl portions can optionally be substituted with a halogen atom or an alkyl, alkyloxy, alkylthio, cyano, trifluoromethyl, carboxy, carboxyalkyl, alkyloxycarbonyl, alkyloxycarbonylalkyl or hydroxyalkyl radical and X denotes a halogen atom, is reacted with a product of general formula:

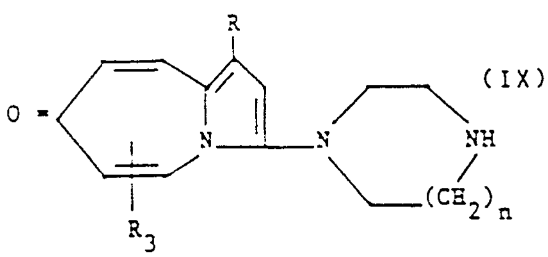

(IX)

in which n equals 1 or 2, $R_3$ is defined as in claim 1, and R is defined as in claim 1 at B), and the product obtained is then isolated and optionally converted to a pharmaceutically acceptable salt.

4. A pharmaceutical composition which contains at least one derivative according to claim 1, in combination with one or more diluents or adjuvants which are compatible and pharmaceutically acceptable.

**Claim for the contracting State: AT**

A process for preparing a new pyrrolo[1,2-a]azepinone derivative of general formula:

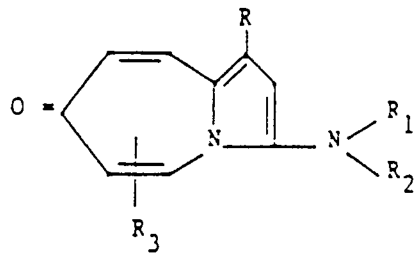

(I)

in which $R_3$ denotes a hydrogen or halogen atom, and

A) either R denotes a phenylthio radical and $R_1$ and $R_2$ form, together with the nitrogen atom to which they are linked, a piperazine ring substituted on the second nitrogen atom with an alkyl radical or alkenyl radical containing 2 to 4 carbon atoms,

B) or R denotes a phenyl radical optionally substituted with one or more halogen atoms or hydroxy, alkyl, alkyloxy or alkylthio radicals, and $R_1$ and $R_2$ form, together with the nitrogen atom to which they are linked, a piperazine or homopiperazine ring substituted on the second nitrogen atom with a hydroxyalkyl radical in which the alkyl portion contains 2 to 4 carbon atoms, an alkenyl radical containing 2 to 4 carbon atoms, an alkynyl radical containing 2 to 4 carbon atoms, or a benzyl or phenethyl radical in which the phenyl portions can optionally be substituted with a halogen atom or an alkyl, alkyloxy, alkylthio, cyano, trifluoromethyl, carboxy, carboxyalkyl, alkyloxycarbonyl, alkyloxycarbonylalkyl or hydroxyalkyl radical, with the understanding that the alkyl radicals and alkyl portions mentioned in the definition above contain, except where otherwise stated, 1 to 4 carbon atoms in a straight or branched chain, as well as the pharmaceutically acceptable salts thereof, wherein,

– for the preparation of a product of general formula (I) in which the symbols are defined as above, with the exception of a product in the formula of which $R_1$ and $R_2$ form, together with the nitrogen atom to which they are linked, a piperazine or homopiperazine ring substituted on the second nitrogen atom with a hydroxyalkyl radical in which the alkyl portion contains 2 to 4 carbon atoms, an alkynyl radical containing 2 to 4 carbon atoms, or a benzyl or phenethyl radical substituted with a cyano radical, the rearrangement is carried out of a product of general formula:

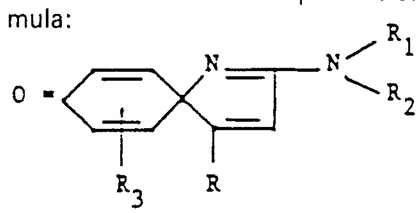

(II)

in which R and $R_3$ are defined as above and $R_1$ and $R_2$ are defined as above with the exception of

forming, with the nitrogen atom to which they are linked, a piperazine or homopiperazine ring substituted on the second nitrogen atom with a hydroxyalkyl radical in which the alkyl portion contains 2 to 4 carbon atoms, an alkynyl radical containing 2 to 4 carbon atoms, or a benzyl or phenethyl radical substituted with a cyano, carboxy, carboxyalkyl or hydroxyalkyl radical, to obtain a product of general formula (I) in which the symbols are defined as above, with the exception of $R_1$ and $R_2$ forming, together with the nitrogen atom to which they are linked, a piperazine or homopiperazine ring substituted on the second nitrogen atom with a hydroxyalkyl radical in which the alkyl portion contains 2 to 4 carbon atoms, an alkynyl radical containing 2 to 4 carbon atoms, or a benzyl or phenethyl radical substituted with a cyano, carboxy, carboxyalkyl or hydroxyalkyl radical, the product obtained is isolated and then, if it is desired to obtain a product of general formula (I) in which R and $R_3$ are defined as above and $R_1$ and $R_2$ form, with the nitrogen atom to which they are linked, a piperazine or homopiperazine ring substituted on the second nitrogen atom with a benzyl or phenethyl radical in which the phenyl portions are substituted with a carboxy or carboxyalkyl radical, the corresponding product of general formula (I), in which R and $R_3$ are defined as above and $R_1$ and $R_2$ form, with the nitrogen atom to which they are linked, a piperazine or homopiperazine ring substituted on the second nitrogen atom with a benzyl or phenethyl radical in which the phenyl portions are substituted with an alkyloxycarbonyl or alkyloxycarbonylalkyl radical, is converted by any known method for converting an ester to an acid or alcohol without affecting the remainder of the molecule, and the product obtained is then isolated and optionally converted to a pharmaceutically acceptable salt, or wherein,

– for the preparation of a product of general formula (I) in which $R_3$ is defined as above and the other symbols are defined as above at B), a product of general formula:

$$R_4X \qquad (VIII)$$

in which $R_4$ denotes a hydroxyalkyl radical in which the alkyl portion contains 2 to 4 carbon atoms, an alkenyl radical containing 2 to 4 carbon atoms, an alkynyl radical containing 2 to 4 carbon atoms, or a benzyl or phenethyl radical in which the phenyl portions can optionally be substituted with a halogen atom or an alkyl, alkyloxy, alkylthio, cyano, trifluoromethyl, carboxy, carboxyalkyl, alkyloxycarbonyl, alkyloxycarbonylalkyl or hydroxyalkyl radical and X denotes a halogen atom, is reacted with a product of general formula:

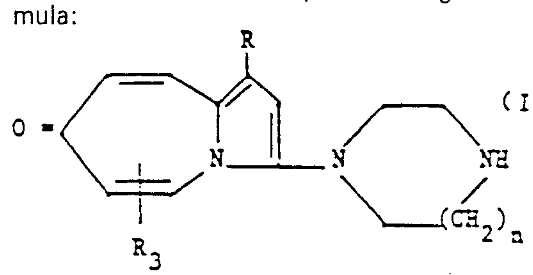

(IX)

in which n equals 1 or 2, $R_3$ is defined as above and R is defined as above at B), the product obtained is then isolated and optionally converted to a pharmaceutically acceptable salt.